Europäisches Patentamt

European Patent Office

Office européen des brevets

(11) Publication number: **0 492 113 A2**

## EUROPEAN PATENT APPLICATION

(21) Application number: **91119254.0**

(22) Date of filing: **12.11.91**

(51) Int. Cl.5: **C12N 15/60**, C12N 9/88, C12N 15/82, A01N 63/00, A01H 1/04

(30) Priority: **27.12.90 US 633210**

(43) Date of publication of application:
**01.07.92 Bulletin 92/27**

(84) Designated Contracting States:
**AT BE CH DE DK ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Hand, John Mark**
**30 River Road, Apt.7E**
**Roosevelt Island, New York 10044(US)**
Inventor: **Singh, Bijay Kumar**
**12 Albermarle Road**
**Hamilton Square, New Jersey(US)**
Inventor: **Chaleff, Roy Scott**
**31 Arvida Drive**
**Pennington, New Jersey 08534(US)**

(74) Representative: **Wächtershäuser, Günter, Dr.**
**et al**
**Tal 29**
**W-8000 München 2(DE)**

(54) Herbicide resistant AHAS deletion mutants.

(57) This invention provides novel nucleic acid sequences encoding herbicide-resistant AHAS enzymes. These sequences contain deletions of one or more amino acids in "conserved" regions of the AHAS molecule. Also disclosed are vectors containing novel sequences, as well as herbicide-resistant plants and plant cells transformed thereby.

This invention relates to novel DNA sequences that encode novel variant forms of acetohydroxy acid synthase enzyme (hereinafter AHAS). The AHAS enzyme is a critical enzyme routinely produced in a variety of plants and a broad range of microorganisms. Normal AHAS function is inhibited by a number of different types of herbicides; however, new AHAS enzymes encoded by the mutant DNA sequences function normally catalytically even in the presence of such herbicides and, therefore, confer herbicide resistance upon the plant or microorganism containing them.

The novel DNA sequences are based on the unexpected observation that deletion of one or more specific amino acids in certain regions of the normal AHAS gene sequence results in a fully functional enzyme, but renders the enzyme resistant to inhibition by a variety of different types of herbicides, including imidazolinones, triazolopyrimidines, and sulfonylureas. The availability of these variant sequences provides a tool for transformation of a variety of different crop plants to herbicide resistance, as well as providing novel selectable markers for use in other types of genetic transformation experiments.

## BACKGROUND OF THE INVENTION

The use of herbicides in agriculture is now widespread. Although there are a large number of available compounds which effectively destroy weeds, not all herbicides are capable of selectively targeting the undersirable plants over crop plants, as well as being non-toxic to animals. Often, it is necessary to settle for compounds which are simply less toxic to crop plants than to weeds. In order to overcome this problem, development of herbicide resistant crop plants has become a major focus of agricultural research.

An important aspect of development of herbicide-resistance is an understanding of how the herbicide works in inhibiting plant growth, and then manipulating the affected biochemical pathway in the crop plant so that the inhibitory effect is avoided while the plant retains normal biological function. One of the first discoveries of the biochemical mechanism of herbicides related to a series of structurally unrelated herbicide compounds, the imidazolinones, the sulfonylureas and the triazolopyrimidines. It is now known (Shaner et al. Plant Physiol. 76: 545-546,1984. U.S. Patent No. 4,761,373) that each of these herbicides inhibits plant growth by interference with an integral cellular enzyme, acetohydroxyacid synthase (AHAS; also referred to as acetolacetate synthase, or ALS). AHAS is required for the synthesis of the amino acids isoleucine, leucine and valine.

The AHAS enzyme is known to be present throughout higher plants, as well as being found in a variety of microorganisms, such as the yeast Saccharomyces cerevisiae, and the enteric bacteria, Escherichia coli and Salmonella typhimurium. The genetic basis for the production of normal AHAS in a number of these species has also been well characterized. For example, in both E. coli and S. typhimurium three isozymes of AHAS exist; two of these are sensitive to herbicides while a third is not. Each of these isozymes possesses one large and one small protein subunit; and map to the IlvIH, IlvGM and IlvBN operons. In yeast, the single AHAS isozyme has been mapped to the ILV2 locus. In each case, sensitive and resistant forms have been identified and sequences of the various alleles have been determined (Friden et. al., Nucl. Acid Res. 13: 3979-3993, 1985; Lawther et al., PNAS USA 78: 922-928, 1982; Squires et al., Nucl. Acids Res 811: 5299-5313, 1983; Wek et al; Nucl. Acids Res 13: 4011-4027, 1985; Falco and Dumas, Genetics 109, 21-35, 985; Falco et al, Nucl. Acids Res 13; 4011-4027, 1985).

In tobacco, AHAS function is encoded by two unlinked genes, SuRA and SuRB. There is substantial identity between the two genes, both at the nucleotide level and amino acid level in the mature protein, although the N-terminal, putative transit region differs more substantially (Lee et al, EMBO J. 7: 1241-1248, 1988). Arabidopsis, on the other hand, has a single AHAS gene, which has also been completely sequenced. Comparisons among sequences of the AHAS genes in higher plants indicates a high level of conservation of certain regions of the sequence; specifically, there are at least 10 regions of sequence conservation. It has previously been assumed that these conserved regions are critical to the function of the enzyme, and that retention of that function is dependent upon substantial sequence conservation.

It has been recently reported (EP 0257993) that mutants exhibiting herbicide resistance possess mutations in at least one amino acid in one or more of these conserved regions. In particular, substitution of certain amino acids for the wild type amino acid at these specific sites in the AHAS sequence have been shown to be tolerated, and indeed result in herbicide resistance of the plant possessing this mutation, while retaining catalytic function. These mutations have been shown to occur at both the SuRA and SuRB loci in tobacco; similar mutations have been isolated in Arabidopsis and yeast.

It has now been unexpectedly discovered that deletions of one or more amino acids within one or more of these "conserved" regions result not only in a functional AHAS enzyme, but also result in herbicide resistance. Sequence conservation normally implies that any change in these regions would not be tolerated and would therefore result in a nonfunctional protein. However, in the present case, it is particularly

EP 0 492 113 A2

surprising that enzyme function is retained, in view of the fact that the deletions not only eliminate an amino acid residue that is a structural component of the enzyme, but also necessarily result in residue shifting, thereby destroying the apparent conservation of the sequence containing the mutation. Thus, novel nucleic acid sequences are provided which are useful in transforming herbicide sensitive plants to herbicide resistant ones. The plants transformed therefore provide the basis for development of novel herbicide resistant plant varieties.

## SUMMARY OF THE INVENTION

The present invention provides novel nucleic acid sequences encoding functional AHAS enzymes insensitive to a variety of herbicides. The sequences in question comprise the deletion of one or more codons encoding a specific amino acid within one or more designated so-called conserved regions in the wild type AHAS molecule. The identity of these sites, and the deletable codons will be discussed in greater detail below. The altered DNA sequences are useful in methods for producing herbicide resistant plant cells, said methods comprising transforming a target plant cell with one or more of the altered sequences provided herein. Alternatively, mutagenesis is utilized to create deletion mutants in plant cells or seeds containing a nucleic acid sequence encoding a herbicide sensitive AHAS. Such plant cells are then passed through tissue culture in order to regenerate plants which possess the herbicide resistant or insensitive trait. The invention thus also encompasses plant cells, plant tissue cultures, adult plants, and plant seeds that possess the deletion mutant nucleic acid sequences and which express functional, herbicide-resistant AHAS enzymes.

The availability of these novel herbicide resistant plants enables new methods of growing crop plants in the presence of herbicides. Instead of growing non-resistant plants, fields may be planted with the resistant plants of the present invention and the field routinely treated with herbicide, with no resulting damage to crop plants. Preferred herbicides for this purpose are imidazolinones, sulfonylureas, and triazolopyrimidines.

The mutant nucleic acids of the present invention also provide novel selectable markers for use in transformation experiments. The nucleic acid sequence encoding a resistant AHAS is linked to a second gene prior to transfer to a host cell, and the entire construct transformed into the host. Putative transformed cells are then grown in culture in the presence of inhibitory amounts of herbicide; surviving cells will have successfully acquired the second gene of interest.

The following definitions should be understood to apply throughout the specification and claims. A "functional" or "normal" AHAS enzyme is one which is capable of catalyzing the first step in the pathway for synthesis of the essential amino acids isoleucine, leucine and valine. A "conserved" sequence or region of AHAS is a series of nucleic acids or amino acids within the AHAS nucleic acid or amino acid sequence which is the same in at least two of the species having the AHAS enzyme. A "wild-type" AHAS sequence is a sequence present in a herbicide sensitive member of a given species. A "resistant" plant is one which produces a normal AHAS enzyme, and which is capable of reaching maturity when grown in the presence of normally inhibitory levels of herbicide. The term "resistant", as used herein, is also intended to encompass "tolerant" plants, i.e., those plants which pheno-typically evidence adverse, but not lethal, reactions to the herbicide.

## BRIEF DESCRIPTION OF THE FIGURES

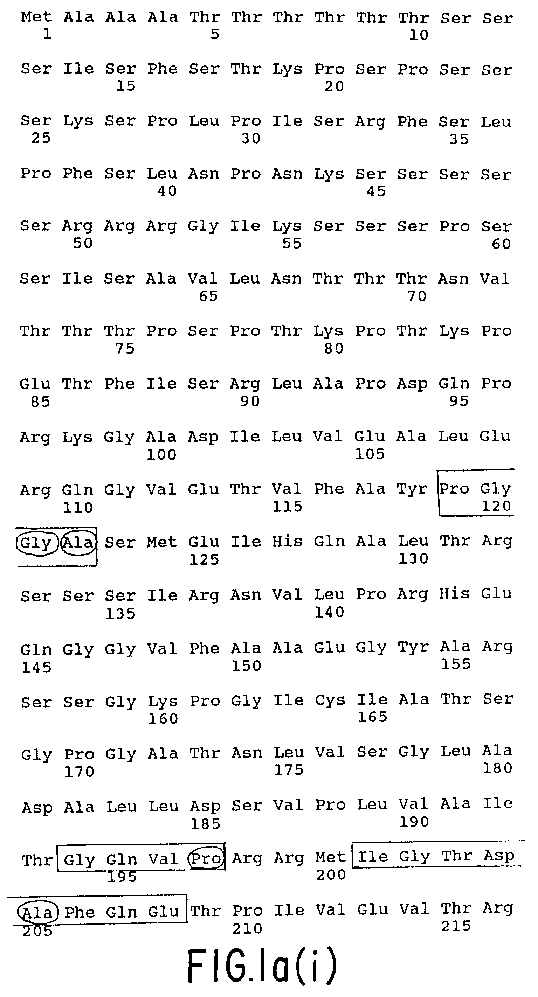

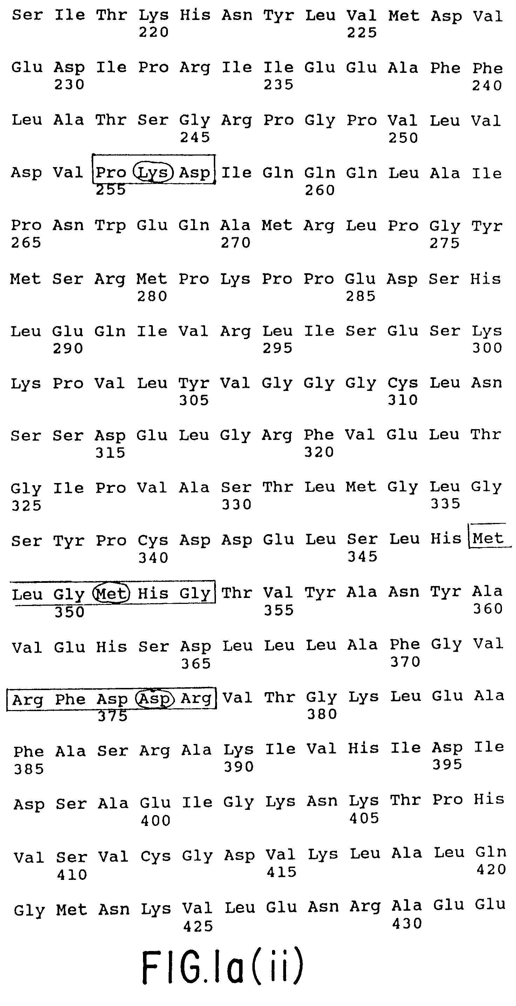

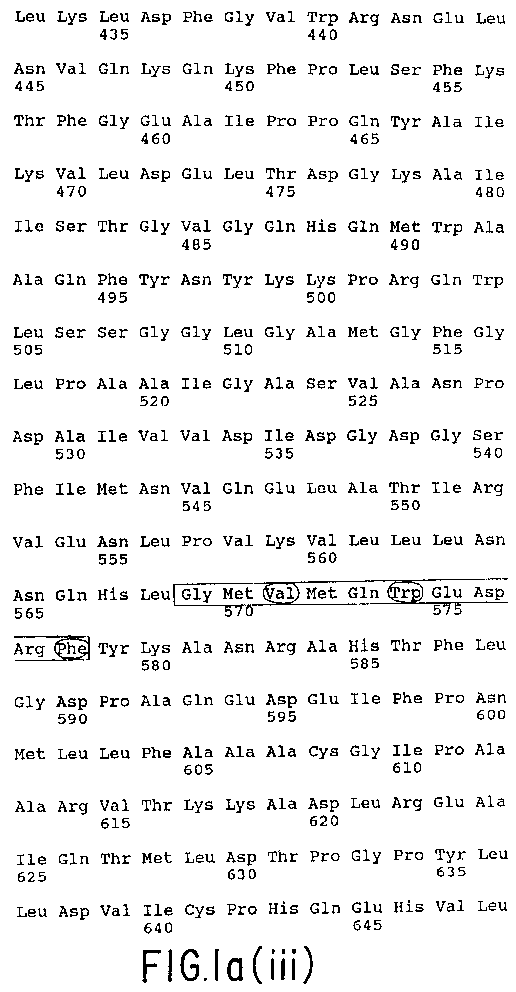

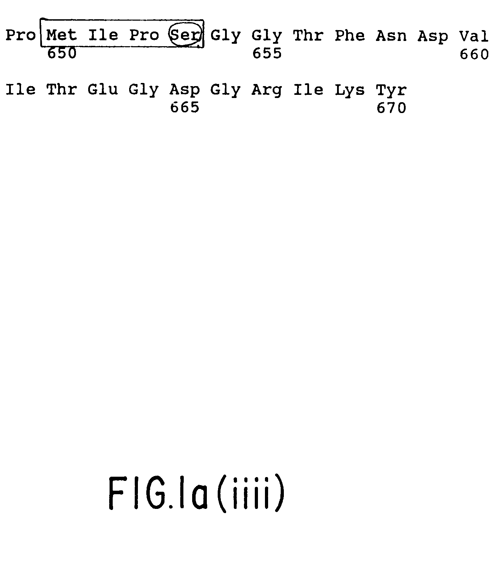

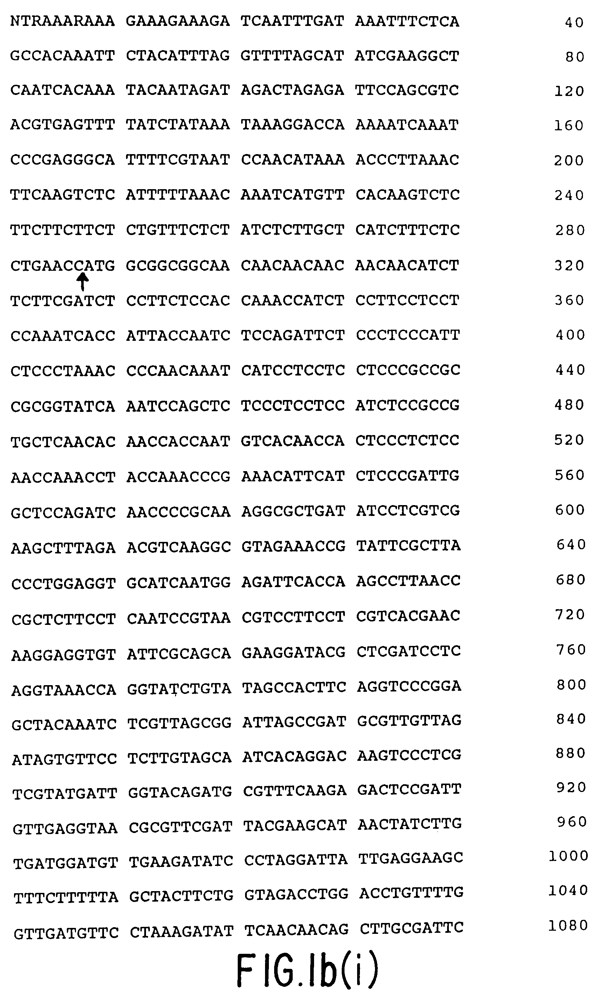

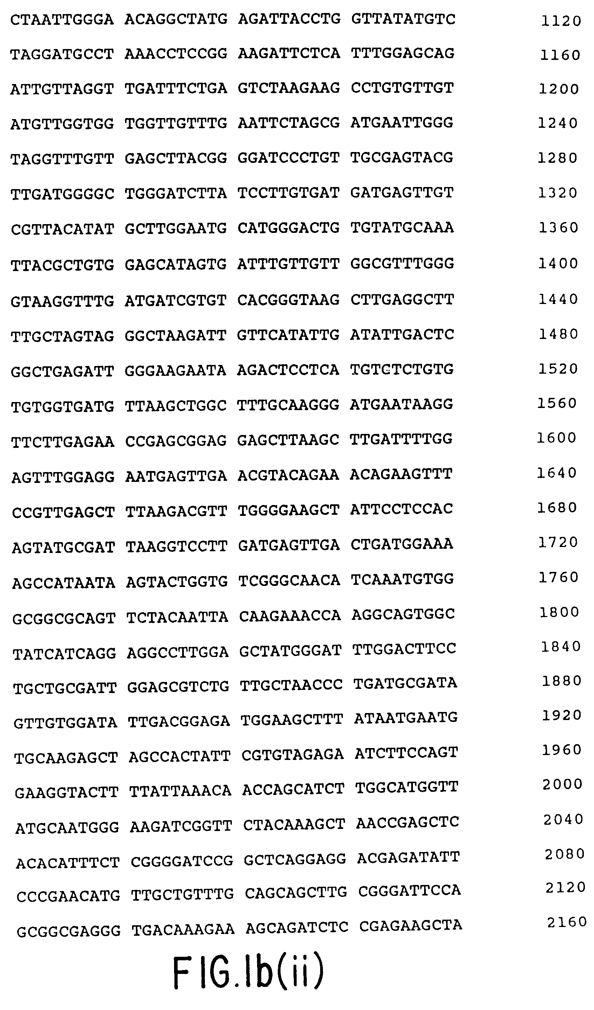

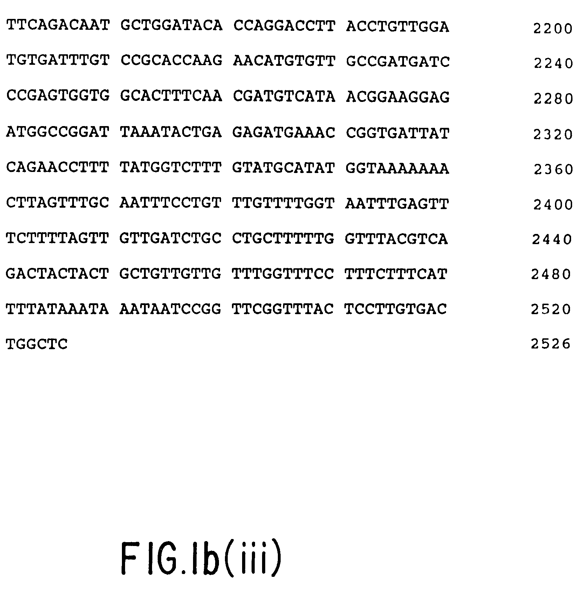

Figures 1a and 1b (Sequence Listings 1 and 2) show, respectively, the amino acid and nucleotide sequences of Arabidopsis wild-type AHAS. In Figure 1a, boxed regions indicate exemplary subsequences in which deletions may be made to produce herbicide resistance. Circled residues identify specific sites where such deletions may be made. In Figure 1b, an arrow indicates the start of the coding region.

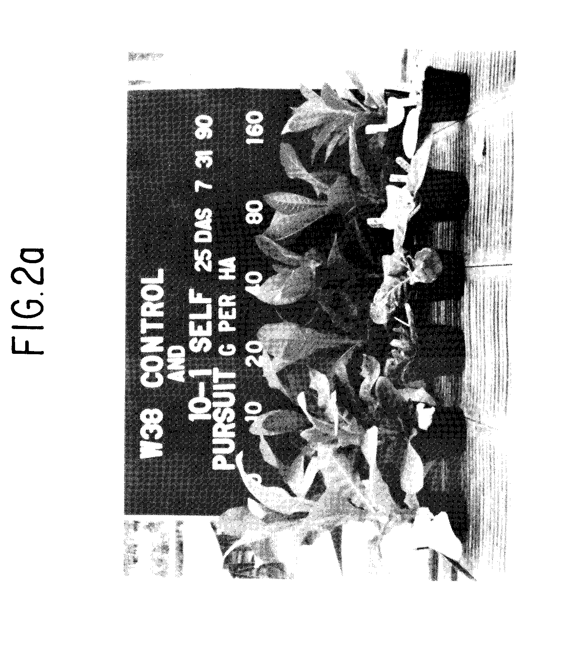

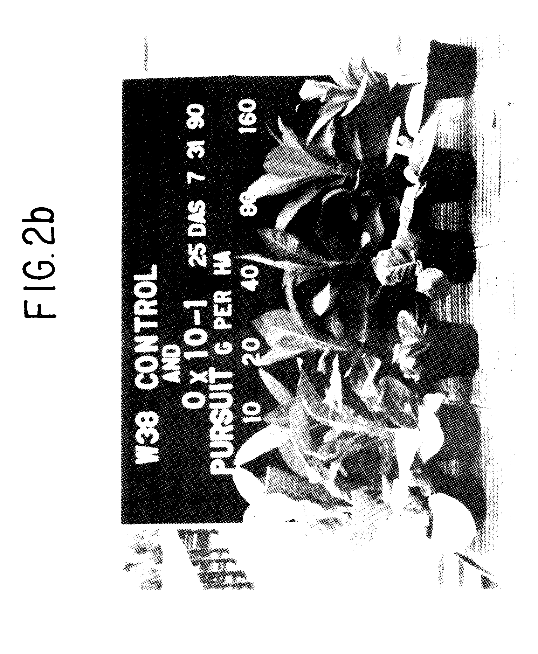

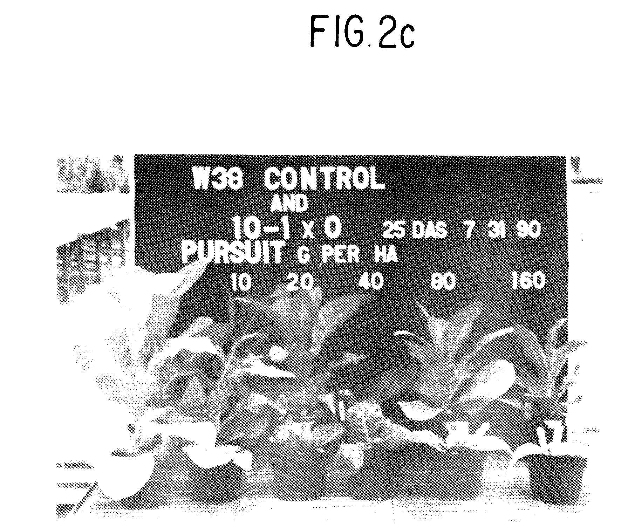

Figure 2(a-c) shows three photographs of progeny (first generation), resulting from a transgenic tobacco plant (10-1) transformed with a construct containing a Trp574 deletion mutation of Arabidopsis AHAS sprayed post-emergence with Pursuit® imidazolinone herbicide at 0, 10, 20, 40, 60, 80 and 160 grams/hectare (g/ha). All three photographs were taken 25 days after spraying. In all photographs, control plants (a wild-type herbicide sensitive tobacco cultivar, Wisconsin 38[W38]) are in the front row.

(a) **W38 Control and 10-1 Self** - W38 represents the control and 10-1 the selfed transgenic tobacco progeny. At 0 g/ha Pursuit®, both the W38 and the 10-1 look similar. At 10 g/ha, the W38 plant is slightly inhibited in its growth as compared to 10-1; however, starting at 20 g/ha, W38 growth is almost completely inhibited. At 80 g/ha, 10-1 plant growth appears to be slightly inhibited and at 160 g/ha, 10-1 plant growth is clearly inhibited.

(b) **W38 Control and 0 x 10-1** - W38 as previously described. 0 x 10-1 represents W38 as the maternal

3

parent (0) and 10-1 as the paternal parent. The results are very similar to that seen in the 10-1 selfed photograph.

(c) **W38 Control and 10-1 x 0** - W38 as previously described. 10-1 x 0 represents 10-1 as the maternal parent and W38 as the paternal parent (0). The results are very similar to that seen in the 10-1 selfed photograph except that in this photo segregation of the herbicide resistance trait can be seen in the 10-1 progeny.

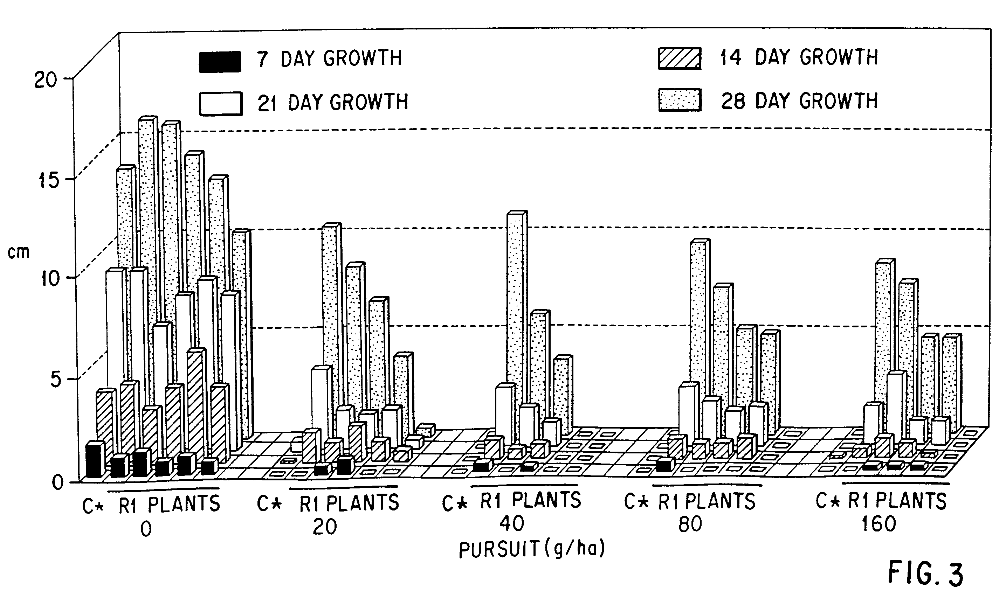

Figure 3 illustrates the effect of Pursuit® postemergent application in tobacco seedling growth, as measured by plant height. C* represents the mean of the control (susceptible parent cultivar) plants, while each subsequent bar represents an individual transgenic progeny.

Figure 4 illustrates the effect of the imidazolinone herbicide Pursuit® on AHAS enzymes derived from an E. coli transformed with a Trp574 deletion [DEL], a Trp574 substitution [SUB] and an E. coli transformed with wild-type [WT] AHAS sequence.

Figure 5 illustrates the effect of the sulfonylurea herbicide Glean® on AHAS enzymes extracted from E. coli transformants. Abbreviations as in Figure 4.

Figure 6 illustrates the effect of the imidazolinone herbicide Pursuit® on AHAS enzymes extracted from E. coli transformants. The enzymes are derived from an E. coli transformed with a Ser653 deletion [DEL], an E. coli transformed with a Ser653 substitution [SUB], and an E. coli transformed with a wild-type [WT] AHAS Sequence.

Figure 7 illustrates the effect of the sulfonylurea herbicide Glean® on AHAS enzymes extracted from E. coli transformants. Abbreviations as in Figure 6.

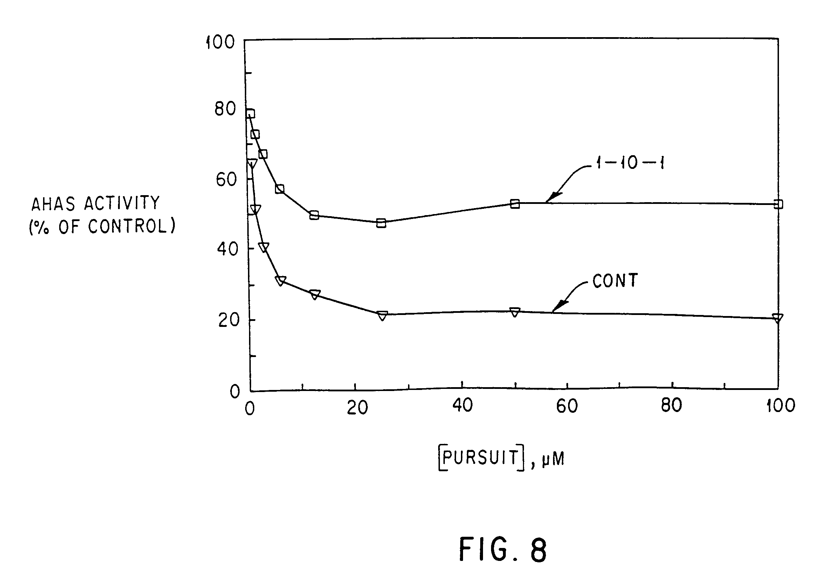

Figure 8 illustrates the effect of the imidazolimone herbicide Pursuit® on an AHAS enzyme derived from a tobacco plant with an Agrobacterium strain containing a mutant Arabidopsis AHAS allele having the Trp574 deletion.

## DETAILED DESCRIPTION OF THE INVENTION

The entire nucleotide sequence of the AHAS gene in a number of organisms including yeast, E. coli, Brassica, Arabidopsis, sugar beet and tobacco has been previously disclosed (Mazur et al., supra. Lee et al., EMBO J. 7:1241, 1988). Moreover, it has been noted that herbicide resistance is associated with one or more mutations within the AHAS sequence; specifically, resistance has been noted to be associated with certain specific amino acid substitutions within the sequence. (EP 259 793; Yadav et al. PNAS USA 83:4418-22, 1986; Sathasivan et al., Nucl. Acids res. 18: 2188, 1990). It has, however, been implied (Hartnett et al., in Managing Resistance To Agrichemicals, Ch. 31, pp. 459-473, American Chemical Society, 1990; EP 259 793) that these regions of conservation in which mutations conferring resistance occur must generally be maintained to conserve enzymatic function.

It has now been unexpectedly discovered that conservation of these sequences is apparently not essential to the catalytic function of the molecule. In the course of development of the present invention, site directed mutagenesis of the Arabidopsis AHAS coding region is utilized to create deletion mutations in which a residue which has previously been shown to be substitutable is then deleted from the sequence. Deletion of a residue clearly destroys the conserved nature of the regions in which they occur. Nonetheless, the deletion mutations thus created all retain AHAS function, while also exhibiting herbicide resistance. Specifically, single deletions of the Trp574, Pro197 and Ser653 residues and double deletions of Pro197 and Ser653 are made to create fully functional, herbicide resistant plants. All the numbering of amino acids herein is based on the Arabidopsis AHAS sequence. However, it will be understood that, throughout the specification and claims, reference to a specific site of deletion in the Arabidopsis sequence is meant to encompass the corresponding sites in the AHAS sequence of any other species having an AHAS gene.

In view of these data, it becomes apparent that conservation of these herbicide-resistance associated regions of the AHAS molecule is not critical for catalytic activity, and that one or more amino acid deletions in one or more "conserved" amino acid sequences can easily be tolerated by the enzyme and further, will confer herbicide resistance to the plant. The present invention encompasses AHAS DNA sequences in which one or more deletions have been made relative to the wild-type sequence, which deletions do not alter the catalytic function of the resulting AHAS enzyme, but which confer herbicide resistance. Such deletion mutation include, but are not limited to, deletion of one or more codons, encoding the so-called "conserved subsequences" as defined in EP 257 793. These are DNA sequences encoding amino acids 119-122, 194-197, 201-208, 255-257, 348-353, 373-377, and 569-578 in Arabidopsis AHAS. Additionally, a further "conserved sequence" in which substitution has been described and in which deletion is useful in bringing about herbicide resistance, is 650-653. Deletion of one codon, more than one codon, and up to all the codons in the "subsequences" noted above, is considered within the scope of the invention. Preferred are codon deletions encoding mutants having a deletion of at least one residue position selected from the group consisting of amino acid 120, 121, 197, 205, 256, 351, 376, 571, 574, 578 and 653. Particularly preferred are sequences encoding deletions at 197, 574 or 653.

Although the above-identified deletions represent regions in which variation is known to be tolerated, it is likely, in view of the substantial "flexibility" of the molecule that other deletions would also be feasible. For example, other apparently "conserved" regions of the AHAS enzyme also exist in addition to those outlined above. While not wishing to be bound by any particular theory, it now appears likely that, rather than being sites connected with catalytic activity of the molecule, and therefore requiring essential structural conservation, most of these conserved regions represent binding sites for the herbicide. Deletion of one or more amino acids at these sites would then logically prevent herbicide binding, and consequently, prevent herbicide interference with AHAS activity. Using this rationale, the available knowledge regarding "conserved" AHAS sequences (See, e.g., Mazur et al., Plant Physiol. 85: 1110-1117, 1987) and known techniques, such as site-specific mutagenesis, it would be a routine matter to design additional deletion mutants likely to have herbicide resistance. Putative mutants can then be screened by growth in the presence of inhibitory amounts of the herbicide of interest, to determine whether or not herbicide resistance has been conferred.

As with all proteins, the functional AHAS enzyme possesses a three-dimensional structure which is the eventual result of the linear arrangement of the component amino acids. Interaction of the side groups of amino acids produces a protein's secondary structure, and further folding results in a protein's tertiary structure. A given protein's specific "architecture" which ultimately results from the amino acid sequence can be critical to the protein's function. Thus, the substitution of an amino acid retains the overall structural integrity of the protein, whereas the deletion of an amino acid effectively destroys this structural integrity and can be expected to significantly alter the protein's three-dimensional structure and, in doing so, alter the function of the molecule. Therefore, in view of the potential damage that can be caused by a deletion, it is particularly surprising that the resultant molecule retains sufficient portions of its three dimensional structure to remain not only catalytically functional, but also to cause herbicide resistance.

Those skilled in the art will recognize that the deletion mutants of the present invention are not limited as to the source of the DNA or enzyme. Although the present experimental design primarily utilizes the Arabidopsis AHAS gene sequence, similar mutations can routinely be made in the AHAS sequence of any organism possessing such a gene, e.g. other higher plants, yeast, E. coli, and other microorganisms. The similarities in the wild-type AHAS gene among all the known sequences are so great as to render it a matter of routine experimentation to create identical mutants in AHAS sequences other than that of Arabidopsis. Alternately, chimaeric genes can be constructed which contain the deletion mutant portion of the Arabidopsis AHAS gene recombined with unaltered portions of the AHAS gene from other sources.

The novel gene types described herein may confer resistance to one, or more than one, type of herbicide. As has already been well established, AHAS is the site of action of several distinct classes of herbicides, namely, imidazolinones, sulfonylureas, triazolopyrimidines, sulfamoylureas and sulfonylcarboxamides. As with herbicide resistance conferred by amino acid substitution, the herbicide resistance conferred by such mutations may be selective to a particular herbicide, or there may be cross-resistance to more than one herbicide. For example, deletions of Trp574 and Ser653 create cross-resistance to both imidazolinones and sulfonylureas. One skilled in the art can, however, easily determine the specificity of any particular mutant by screening separately in the presence of, e.g., imidazolinones only, or sulfonylureas only, and isolating surviving plants. Cross resistance can be determined by growth in the presence of more than one class of herbicide. Types of herbicides with which the present invention is useful are described, for example, in U.S. Patent No. 4,188,487; 4,201,565; 4,221,586; 4,297,128; 4,554,013; 4,608,079; 4,638,068; 4,647,301; 4,650,514; 4,698,092; 4,701,208; 4,709,036; 4,752,323; 4,772,311; and 4,798,619; U.S. Pat. Nos. 4.127,405; 4,435,206; 4,424,703; 4,417,917; 4,398,939; 4,394,506; 4,391,627; 4,383,113; 4,378,991; 4,372,778; 4,371,391; 4,370,480; 4,370,479; 4,369,320 (sulfonylureas).

At this time, AHAS has been demonstrated to be not only present in a wide variety of plants, but has also been shown to be a critical site determining herbicide sensitivity in a broad range of essentially unrelated plants, e.g. corn, Brassica, tobacco, flax, Arabidopsis, and sugar beet (Stougaard et al., Mol. Gen. Genet. 219: 413-420, 1989; Jordan & McHughen, J. Plant Physiol. 131: 333-338, 1987; McHughen, Plant Cell Reports 8: 445-449, 1989). As noted above, then, it is possible to create the relevant mutation directly in the plant of interest by known mutagenic techniques. (See, for example, Maniatis, et. al., Molecular Cloning, A Laboratory Manual, Cold Spring Harbor Laboratory, 1982; and Example II, infra). However, it will frequently be more convenient to create transformed plants with a known and isolated DNA sequence comprising the requisite deletions, such as the Arabidopsis deletion mutants described in the present case. Plasmids containing deletion mutants at positions 574 and 653 have been deposited with the American

5

Type Culture Collection, Rockville, MD, on December 6, 1990 as, respectively, accession numbers ATCC 68488 and 68489.

Isolated AHAS DNA sequences of the present invention are useful to transform target crop plants, and thereby confer herbicide resistance without the necessity for mutagenesis. A broad range of techniques currently exist for achieving direct or indirect transformation of higher plants with exogenous DNA, and any method by which the novel sequence can be incorporated into the host genome, and stably inherited by its progeny, is contemplated by the present invention. A detailed description of one such method is provided in the following examples.

Indirect transformation of plant cells can be achieved by the use of vectors. A common method of achieving transformation is the use of Agrobacterium tumefaciens to introduce a foreign gene into the target plant cell. For example, in the present case, the mutant AHAS sequence is inserted into a plasmid vector containing the flanking sequences in the Ti-plasmid T-DNA. The plasmid is then transformed into E. coli. A triparental mating among this strain, an Agrobacterium strain containing a disarmed Ti-plasmid containing the virulence functions needed to effect transfer of the AHAS containing T-DNA sequences into the target plant chromosome, and a second E. coli strain containing a plasmid having sequences necessary to mobilize transfer of the AHAS construct from E. coli to Agrobacterium is carried out. A recombinant Agrobacterium strain, containing the necessary sequences for plant transformation is used to infect leaf discs. Discs are grown on selection media and successfully transformed regenerants are identified. The recovered plants are resistant to the effects of herbicide when grown in its presence. Other plant vectors, such as plant viruses, also provide a possible means for transfer of exogenous DNA.

Direct transformation of plant cells, instead of the use of vectors, can also be employed. Typically, protoplasts of the target plant are placed in culture in the presence of the DNA to be transferred, and an agent which promotes the uptake of DNA by protoplast is absorbed on their surfaces. Useful agents in this regard are polyethylene glycol or calcium phosphate.

Alternatively, DNA uptake can be stimulated by electroporation. In this method, an electrical pulse is used to open temporary pores in a protoplast cell membrane, and DNA in the surrounding solution is then drawn into the cell through the pores. Similarly, microinjection can be employed to deliver the DNA directly into a cell, and preferably directly into the nucleus of the cell.

In each of the foregoing techniques, transformation occurs in a plant cell in culture. Subsequent to the transformation event, plant cells must be regenerated to whole plants. Techniques for the regeneration of mature plants from callus or protoplast culture are now well known for a large number of different species (see, e.g., Handbook of Plant Cell Culture, Vols. 1-5, 1983-1989 McMillan, N.Y.) Thus, once transformation has been achieved, it is within the knowledge in the art to regenerate mature plants from the transformed plant cells.

Alternate methods are also now available which do not necessarily require the use of isolated cells, and therefore, regenerative techniques, to achieve transformation. These are generally referred to as "ballistic" or "particle acceleration" methods, in which DNA coated metal particles are propelled into plant cells by either a gunpowder charge (Klein et al., Nature 327: 70-73, 1987) or electrical discharge (EPO 270 356). In this manner, plant cells in culture or plant reproductive organs or cells, e.g. pollen, can be stably transformed with the DNA sequence of interest.

The present invention can be applied to transformation of virtually any type of plant, both monocot and dicot. Among the crop plants for which transformation to herbicide resistance is contemplated are corn, wheat, rice, millet, oat, barley, sorghum, alfalfa, sugar beet, Brassica species, tomato, pepper, soybean, tobacco, melon, squash, potato, peanut, pea, cotton, or cacao. The novel sequences may also be used to transform ornamental species, such as rose, and woody species, such as pine.

The novel sequences of the invention also is useful as selectable markers in plant genetics studies. For example, in plant transformation, sequences encoding herbicide resistance could be linked to a gene of interest which is to be used to transform a target herbicide - sensitive plant cell. The construct comprising both the gene of interest and the herbicide resistant sequence are introduced into the plant cell, and the plant cells are then grown in the presence of an inhibitory amount of the herbicide. Plant cells surviving such treatment presumably have acquired the resistance gene as well as the gene of interest, and therefore, putative transformants are readily identifiable.

The invention is further illustrated by the following non-limiting examples.

## EXAMPLE I

### Isolation of a Genomic Clone Encoding Arabidopsis AHAS

A 2.1Kb EcoRI fragment encompassing the promoter, transit peptide and a portion of the mature coding region of Arabidopsis AHAS is $^{32}$P-labelled by nick translation (Maniatis et al., Molecular Cloning: A Laboratory Manual, Cold Spring Harbor Laboratory, Cold Spring Harbor, N.Y., 1982) and utilized as a hybridization probe to screen a genomic library prepared from Arabidopsis thaliana genomic DNA (Clontech, Palo Alto, CA). The filters are hybridized for 24 hr. at 42°C in 6xSSC, 5 x Denhardt's solution, 50mM sodium phosphate, pH7.2, 0.1% SDS and 100ug/ml of denatured salmon sperm DNA. Filters are washed several times at 60°C in 1xSSC and 0.1%SDS. Six recombinant bacteriophage (A22, A31, A42, A52, A72, A83) were classified as putative positives. These bacteriophage are plaque purified and infected at low multiplicity with the E. coli strain K802 in liquid NZY broth (NZCYM is 10 g NZ amine, 5 g NaCl, 5 g yeast extract, 1 g casamino acids, 2 g MgSO$_4$-7H$_2$O and 10ml of 1M Tris-HCl, pH7.2) as described by Maniatis, et al, supra. Liquid cultures are incubated overnight with constant shaking (300 rpm) at 37°C. The 50ml suspension is brought to IM NaCl and 8% PEG by the addition of solid NaCl and solid polyethylene glycol. The suspension is incubated overnight at 4°C to precipitate the bacteriophage. The bacteriophage are pelleted via centrifugation at 18,000 x g for 20min. The pellet is resuspended in 2ml of TM buffer (10mM tris-HC1, pH 7.5; 10mM MgC1$_2$), layered upon a CsC1 step gradient (4.8M CsC1, 4.0M CsC1 and 3.2M CsCl) and centrifuged at 50,000 x g for 1 hr in a SW60 swinging bucket rotor. The phage band is removed from the 4.0/3.2M CsCl interface to a 1.5ml Eppendorf tube and the bacteriophage lysed by adding 1 volume of formamide and incubating at room temperature for 30 minutes. DNA is recovered by bringing the solution to 10m MTris-HC1, pH 8.0 and 1mM Na$_2$- EDTA and precipitating the DNA via the addition of 2 volumes of 100% ethanol. Bacteriophage DNA is recovered via centrifugation, washed with 70% ethanol and resuspended in TE buffer (TE buffer is 10mM Tris-HC1, pH 7.5; 1mM Na2-EDTA). The purified DNA is routinely extracted one to several times with phenol:chloroform:isoamyl alcohol (24:24:1), ethanol precipitated, and resuspended in 50-100ul of TE buffer prior to digestion with various restriction enzymes.

DNA preparations from the six recombinant phage are digested with various restriction enzymes and resolved by electrophoresis through a 1% agarose gel. The DNA is transferred to nitrocellulose (Southern) and hybridized (24 hr. at 60°C in 2xSSC, 5 x Denhardt's solution, 50mM sodium phosphate, pH7.2, 0.1% SDS and 100ug/ml of denatured salmon sperm DNA) against a radiolabeled 900 bp Nco1/EcoRI fragment representing the 3' region of the 2.1Kb EcoRI fragment (the transit peptide and the 5' portion of the mature coding region). The filter is washed twice at 65°C in 0.5xSSC and 0.1%SDS. The 900 bp Nco1/EcoRI fragment hybridizes to a 5.5Kb Xba1 and 2.1 EcoRI fragment of both A42 and A52. These data are in agreement with published information concerning the cloning and characterization of the genomic sequence encoding Arabidopsis AHAS (Mazur et al., Plant Physiol, 85: 1110-1117, 1987).

The bacteriophage A52 is chosen for further analysis. The 5.5 Kb XbaI fragment of A52 is isolated from a 1% agarose gel and purified through an IBI electroelution device as suggested by the manufacturer. The 5.5Kb Xba1 fragment is ethanol precipitated and resuspended in ddH$_2$0. These fragments are ligated to Xbal digested-pSK(-) plasmid DNA (purchased from Stratagene, La Jolla, CA). The fragments are ligated for 16-24 hours at 14°C in a 20ul volume as described by Maniatis, et. al., supra. Following incubation, the 20ul reaction volume is diluted to 100ul with 100mM Tris-HC1, pH7.2. Twenty five ul of this sample is diluted further to 100 ul with 100mM Tris-HC1, pH7.2 and incubated with 100ul of competent HB101 cells [prepared exactly as described by Morrison (Meth. Enzymol. 68: 326 - 331, 1979)]. The plasmid/E. coli transformation mix is incubated on ice for 20 minutes; transferred to 42°C for 2 minutes; room temperature for 10 minutes; followed immediately by the addition of 1 ml of LB broth (LB broth is 10 g bacto-tryptone; 5 g yeast extract; 5 g NaC1 and 10 ml 1 M Tris-HC1, pH 7.2 per liter) and further incubation at 37°C for 20 minutes. DNA plasmid cloning vehicles used in this work carry either the gene conferring resistance to ampicillin or kanamycin. Therefore, 200 ul of each transformation mix is plated directly onto either LB$_{amp}$ or LB$_{kan}$ plates (100 ug/ul of either respective antibiotic) and incubated at 37°C for 12-20 hr, depending on colony size.

Colonies are transferred to 3ml of LB$_{amp}$ or LB$_{kan}$ and incubated overnight at 37°C with constant shaking. Small scale plasmid DNA preparations are prepared by minor modifications of the alkaline lysis method described by Maniatis, et al. supra. Routinely, 400 ul of the 3ml overnight culture is pelleted in a 1.5ml Eppendorf tube and resuspended in 150 ul of GTE solution (GTE solution is 50mM glucose; 25mM Tris-HC1, pH 8.0 and 10mM Na2-EDTA) and incubated on ice for 20 minutes, followed by the addition of 300 ul alkaline-SDS solution and 200 ul of 5M K$^+$/3M OAc at the prescribed time intervals. The final DNA pellet is resuspended in 40ul of 50ug/ul RNAse A in TE buffer. Fifteen ul of plasmid DNA is removed for restriction digestion. Two plasmids, pAC301 (5'-->3') and pAC302 (3'-->5'), represent constructions containing the 5.5Kb Xbal fragment ligated to the Xbal site of pSK(-). Further restriction analysis of pAC301 and pAC302 confirm the presence of the promoter region, transit peptide, mature coding region and 3'non-translated regions of the genomic sequence encoding Arabidopsis AHAS (Mazur et al., Supra).

**EXAMPLE II**

**Oligonucleotide site directed mutagenesis of the Arabidopsis AHAS coding region**

    **(a) Preparation of single stranded template of pAC301** - The plasmid pAC301 is transformed into the E. coli strain, XL-1 (Stratagene). Single stranded template of pAC301 is generated from this pSK(-) "phagemid" based construction by following the manufacturers protocol. Routinely, a 3ml $SB_{amp}$ overnight culture is added to a sterile flask containing 50ml of $SB_{amp}$ (SB is 35 g bacto-tryptone, 20 g yeast extract, 5 g NaC1 and 10 ml of 1M Tris-HC1, pH 7.5 per liter) and incubated at 37°C with constant shaking (300 rpm) until the culture reached an OD600 of 0.3. At this point the culture is inoculated with the helper phage, R408 ($1 \times 10^{10}$ phage) and vigorous 10° shaking at 300 rpm at 37°C was continued overnight. The bacteria are pelleted via centrifugation at 10,000 x g for 10 minutes. The bacterial pellet is discarded and the supernatant (approximately 45 ml) containing the single stranded pAC301 template is stored at 4°C until use. The supernatant is routinely used for up to one month for single stranded phagemid preparations. Small scale preparations (1.2ml) of single stranded pAC301 phagemid DNA are prepared essentially as described by the manufacturer (Stratagene). The phage are precipitated by adding 300 ul of a 3.5M $NH_4Ac$, pH 7.5; 20% PEG solution. The solution is mixed well in a 1.5 ml Eppendorf tube and incubated at room temperature for 20 minutes. The single stranded phage are pelleted by centrifugation for 20 minutes in an Eppendorf microcentrifuge. The supernatant is decanted, the pellet dried and eventually resuspended in 300 ul of TE buffer. The phage are lysed by adding 200 ul of phenol:chloroform:isoamyl alcohol (24:24:1) and vortexing for 1 minute. This step is repeated once more and followed by two more extractions with chloroform:isoamyl alcohol (24:1). The final aqueous phase is ethanol precipitated (one-tenth volume 5M $K^+$/3MOAC and two volumes of 100% ethanol), washed with 70% ethanol, resuspended in 20ul of TE buffer and transferred to a fresh 1.5ml Eppendorf tube.

    **(c) Preparation of the oligonucleotide** - All oligonucleotides are purchased from New England Biolabs. Oligonucleotides are utilized to introduce (1) an amino acid substitution at the Trp574 residue of the Arabidopsis AHAS coding region (Trp --> Leu) and (2) an amino acid deletion at the Trp574 amino acid residue.

```
Trp574 --> Leu substitutions
                    V    M    Q    W    E    D    R
wild type 5'-GTT ATG CAA TGG GAA GAT CGG-3'

                    V    M    Q    L    E    D    R
mutant    5'-GTT ATG CAA TTG GAA GAT CGG-3'(21-mer)


Trp574 deletion
                    V    M    Q    W    E    D    R
wild type 5'-GTT ATG CAA TGG GAA GAT CGG-3'

                  V    M    Q         E    D    R
mutant    5'-G GTT ATG CAA --- GAA GAT CGC- 3'
                            (21-mer)
```

    Two hundred nanograms of each oligonucleotide is subjected to a kinase exchange reaction prior to hybridization to pAC301 single stranded template. A 40 ul reaction volume included 50mM Tris-HC1, pH7.5; 10mM MgCl2, 50mM DTT, 0.1mM spermidine and 0.1mM Na2-EDTA. The reaction is initiated via the addition of 10 units of T4 polynucleotide kinase (Pharmacia) and is incubated at 37°C for 30 minutes. The reaction is terminated by bringing the reaction mixture to 90°C for 3 minutes. One-half of the kinase reaction is added to the entire 20 ul pAC301 single stranded prep and is incubated at 65°C for 10 minutes to promote hybridization. To this 40 ul kinase/oligonucleotide mix is added 6 ul of 1mM dNTP's, 6 ul of 10 x ligase buffer (500mM Tris-HC1, pH7.5; 70 mM MgC1$_2$ and 10mM DTT), 2 ul 10mM rATP, 5 units of Klenow DNA polymerase (Pharmacia), 8 units of DNA ligase (Stratagene) and 4ul ddH$_2$0. The polymerase/ligation reaction is incubated at room temperature for 3 hr. One-half of the mix is used to transform competent E. coli XL-1 cells. The transformation mix is spread to LB$_{amp}$ plates and incubated overnight at 37$^o$C. Transformants are restreaked to fresh LB$_{amp}$ plates in a grid fashion. Colony screening by hybridization was performed exactly as described by Stratagene Technical Service (June 1988/pBSII Exo/Mung Bean DNA Sequencing System). Both oligonucleotides are $^{32}$P-labeled by a kinase exchange reaction (300 ng of oligonucleotide in the presence of 40 uCi of σ$^{32}$P-ATP utilizing kinase conditions described previously). Unincorporated $^{32}$P-ATP is removed by electrophoresing the kinase reaction through a 20% acrylamide/7M urea gel. The radioactive band corresponding to the 21-mer oligonucleotide is cut out of the gel with a razor blade and eluted either by (1) the crush and soak method of Maxam and Gilbert (PNAS USA 74: 560-566, 1977) or (2) electroelution through an IBI electroelution device. The purified, radiolabeled oligonucleotides are hybridized to the nitrocellulose colony lifts under non-stringent hybridization conditions (37$^o$C for 24 hrs. in 6xSSC, 5 x Denhardt's, 50mM Na-P, pH7.2 and 500 ug/ul of calf thymus DNA). Filters are washed once at room temperature in 6xSSC and 50 mM Na-P, pH 7.2 and again at 37$^o$C in 6xSSC and 50mM Na-P, pH 7.2. Finally, the filters are washed twice at 60$^o$C for 15 minute intervals in 3M tetramethylammonium chloride, 50mM Tris-HC1, ph 7.5, 2mM Na$_2$-EDTA and l mg/ml of SDS. A 21-mer oligonucleotide with a perfect base to base hybridization match (i.e., mutant oligo to mutant phagemid) is not washed free of the phagemid DNA at 60$^o$C, whereas a slight mismatch (i.e., mutant oligo to wild type phagemid) is washed free. Putative positives are streaked from the original LB$_{amp}$ plate to a fresh LB$_{amp}$ plate. Ten transformants from each restreaked putative positive are then restreaked in a grid pattern and the colony hybridization is repeated. Secondary positives are then confirmed via DNA sequence analysis through the Trp574 coding region. (Sanger et al., PNAS VSA 74: 5463-5467, 1977). Two positive mutants, pAC324 (Trp574 -- > Leu substitution) and pAC325 (Trp574 deletion) were selected for further analysis.

## EXAMPLE III

### Agrobacterium Mediated Transformation of Tobacco

**(a) Construction of vectors** - Both vectors and the Agrobacterium strain are purchased from Clontech. The plasmid pBIN19 is an E. coli/Agrobacterium binary shuttle vector that possesses both the left and right border sequences of the Ti-plasmid T-DNA, a polylinker, an RK2 bacterial origin of replication functional in both E. coli and Agrobacterium, and a gene conferring resistance to kanamycin (Bevan, Nucl. Acids Res. 12: 8711-8721, 1984) The plasmids pAC324 and pAC325 are digested with Xbal and electrophoresed through a 1% agarose gel. The 5.5Kb Xbal fragment containing the respective mutation of pAC324 and pAC325 are isolated as previously described. The plasmid pBIN19 is digested with Xbal and incubated in a separate ligation reaction with the 5.5Kb Xbal fragment from pAC324 and pAC325. The entire ligation mix is transformed into E. coli XL-1 cells and positive transformants are chosen via a blue/white color selection on LB$_{aix}$ plates (LB plates with 100 ug/ul ampicillin, 80 ug/ml X-Gal and 20mM IPTG). Small scale plasmid preparations, restriction digestion analysis and agarose gel electrophoresis to confirm positive transformants are performed as described previously. Of the four pBIN19 based plasmid constructions, pAC348-pAC349 (both orientations of the 5.5Kb Xbal fragment containing the Trp574-->Leu substitution in pBIN19) and pAC350-pAC351 (both orientations of the 5.5Kb Xbal fragment containing the Trp574 deletion in pBIN19), one construction from each respective mutation is chosen for transformation of tobacco.

### (b) Mobilisation of pAC348 - pAC351 into Agrobacterium

The plasmid pRK2013 is a conjugative plasmid that contains trans acting sequences required to mobilize pBIN19 based constructions from E. coli into the disarmed Agrobacterium strain, LBA4404 (pAL4404). This Agrobacterium strain is resistant to streptomycin and contains the disarmed Ti plasmid pAL4404 (Ooms et al., Plasmid 7: 15-29, 1982). This Ti plasmid contributes the transacting virulence functions necessary to facilitate transfer of the pBIN19 based T-DNA region into the chromosome of tobacco. The triparental mating of the pBIN19 based vectors, the conjugative plasmid (pRK2013), and the Agrobacterium strain (LBA4404), are carried out essentially as described by Bevan (Nucl. Acids Res. 12: 8711-8721, 1984. The plasmids pAC348-351 (Kan$_r$) and pRK2013 (Kan$_r$) are transformed into competent E. coli HB101 or XL-1 cells as described previously. Transformants containing each plasmid are inoculated into 3ml LB$_{kan}$ broth cultures and incubated at 37$^o$C with constant shaking. The Agrobacterium strain, LBA4404, is inoculated into AB$_{strp}$ media (20 x AB is 20g NH4C1, 6g MgS0$_4$-7H$_2$0, 3g KC1, 60g K$_2$HPO$_4$,

$20g$ $NAH_2PO_4$, $3g$ $CaCl_2$ and $50mg$ $FeSO_4 \cdot 7H_2O$. Glucose is added to a 1x stock to a final concentration of 0.5x) and incubated with constant shaking at $28^{\circ}C$ for 36-48 hr. The triparental mating is initiated by combining 1 ml of each of the three cultures (a pBIN19 based construct, pRK2013 and LBA4404) into a sterile tube and continuing incubation at $28^{\circ}C$ for 1 hr. The culture is concentrated via vacuum onto a 0.45uM filter and incubated overnight at $28^{\circ}C$ on an NB plate (NB is 4g of Difco nutrient broth powder and 10ml of 1M Tris-HCl, pH 7.2). The filter is placed in 2ml of fresh AB media and rotated slowly for 1 hr. A dilution series is spread onto $AB_{strp/kan}$ plates and incubated at $28^{\circ}C$ for 3-4 days. Only Agrobacterium - (strp$_r$) carrying the pBIN19 based construct (kan$_r$) grow on $AB_{strp/kan}$ media. Single colonies from $AB_{strp/kan}$ plates are inoculated into $AB_{strp/kan}$ broth and incubated at $28^{\circ}C$ with constant shaking for 36-48 hr. These cultures are restreaked to $AB_{strp/kan}$ plates, incubated at $28^{\circ}C$ for 3-4 days and stored at $4^{\circ}C$ until use.

**(c) Agrobacterium-Mediated Transformation of Tobacco**

pAC348 and pAC351 are inoculated into 50ml of $AB_{kan}$ and incubated with constant shaking at $28^{\circ}C$ for 36-48 hr. Bacteria are pelleted by centrifugation at 2500 rpm for 10 minutes in a Damon/IEC table top centrifuge. The bacterial pellet is resuspended in 5ml of BAT media (BAT media is lx MS salts (Murashige and Skoog, Plant Physiol. 15: 473-497, 1962) 1x $B_5$ vitamins [100x $B_5$ is 10mg myo-inositol, 100mg nicotinic acid, 100mg of pyridoxine-HCl and 1000mg Thiamine-HCl per 1 liter], 3% sucrose, 5uM 6-benzylaminopurine at pH 5.7). Young greenhouse grown Wisconsin 38 tobacco leaves are cut into 2 longitudinal sections and soaked in warm water containing Ivory hand soap. The leaf sections are washed in sterile distilled water, soaked in a 10% Clorox + Tween 20 solution for 10 minutes with stirring and rinsed 3x with $ddH_2O$. Discs are cut from the leaf sections using either a cork borer or hole-punch. These discs are placed in a 50ml tube containing the resuspended Agrobacterium culture. The suspension is mixed gently for 5' minutes and blotted onto a sterile paper towel and cultured onto BAT media plates (100 x 20mm plates with approximately 10 discs per plate). The plates are incubated at $25^{\circ}C$ under fluorescent lighting for 48hr. The discs are then transferred to selection media (BATCK; BAT media plus carbenicillin and kanamycin) and returned to the initial incubation conditions until the formation of shoots. Kanamycin resistant shoots are transferred to OTCK (rooting) selection media (OTCK media is BATCK media minus 6-benzylaminopurine) in (GA7) Magenta boxes and the previously described incubation conditions were continued. When kanamycin resistant shoots form an appreciable root system (at least 3 roots with lengths longer than 1cm), the plants are transferred to soil. Briefly, the agar based shoot is removed from the GA7 box, the agar is carefully washed away with warm tap water and the shoots are transferred to metromix in peat pots in GA7 vessels. The plants are placed in the greenhouse and allowed to harden off. A total of 5 kanamycin resistant shoots containing pAC348 and 9 kanamycin resistant shoots containing pAC350 are transferred to soil. Approximately 10-14 days later, the peat pots are transplated to larger pots.

| pAC348 Transformant #: | pAC351 Transformant #: |
|---|---|
| 1) 19-1 | 1) 10-1 |
| 2) 33-1 | 2) 20-1 |
| 3) 33-2 | 3) 20-2 |
| 4) 46-1 | 4) 20-3 |
| 5) 46-2 | 5) 27-1 |
|  | 6) 27-2 |
|  | 7) 27-3 |
|  | 8) 30-1 |
|  | 9) 42-4 |

**EXAMPLE IV**

**Determination and Characterization of Mutants Conferring Herbicide Resistance**

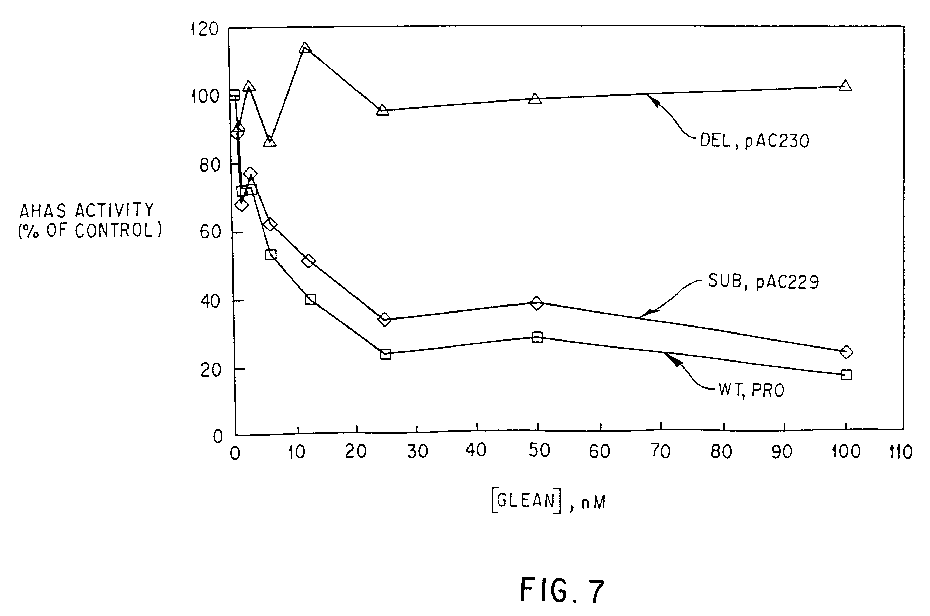

**AHAS enzyme assay (leaf tissue)** - Shortly after transfer to the greenhouse, extracts each of the kanamycin resistant transformants are prepared and assayed for insensitivity to the imidazolinone Pursuit® herbicide. Acetohydroxyacid synthase is extracted and assayed as described in the previous section. Two kanamycin resistant transformants (33-1 and 46-2) which contain the Trp574-->Leu substitution mutant allele of the Arabidopsis AHAS allele exhibit AHAS activity in the presence of Pursuit® herbicide. In addition, AHAS activity of one kanamycin resistant deletion transformant (10-1) exhibits insensitivity to the addition of Pursuit® herbicide (Figure 7). This Trp574 deletion transformant is selfed and backcrossed to Wisconsin 38. Progeny from 10-1 exhibit tolerance to post-emergence applications of Pursuit® herbicide 4-8x greater than the concentration lethal to control tobacco plants.

Figures 2(a)-(c) show photographic comparisons of the phenotypes of the crosses and controls discussed above. These observations indicate inheritance of resistance observed in the initial AHAS assay from transformed leaf tissue, and expression of this trait at the whole plant level.

In a more quantitative assay, seed of selfed progeny of the transgenic tobacco plant (10-1) containing the Trp574 deletion mutation of Arabidopsis AHAS as well as seeds of the susceptible parental cultivar "Wisconsin 38" are planted in Metro Mix 350™ in 5" Azalea pots. The seedlings are thinned to the single most vigorous seedling after 2 days. Eleven days later, these plants are sprayed postemergence with Pursuit® at 0, 10, 20, 40, 80 and 160 g/ha. Five plants of each tobacco type (transgenic and control) are sprayed per herbicide rate. Tween 20™ is added at 0.25 v/v to the herbicide solutions prior to spraying. The herbicide is applied with a laboratory belt sprayer at a rate of 400 L/ha at a distance 18" above the plants with a belt speed of 8.2 sec/rev and sprayer nozzle #65015E. Plant heights are measured 1, 2, 3, and 4 weeks after treatment, plant fresh weight data is collected at four and one-half weeks.

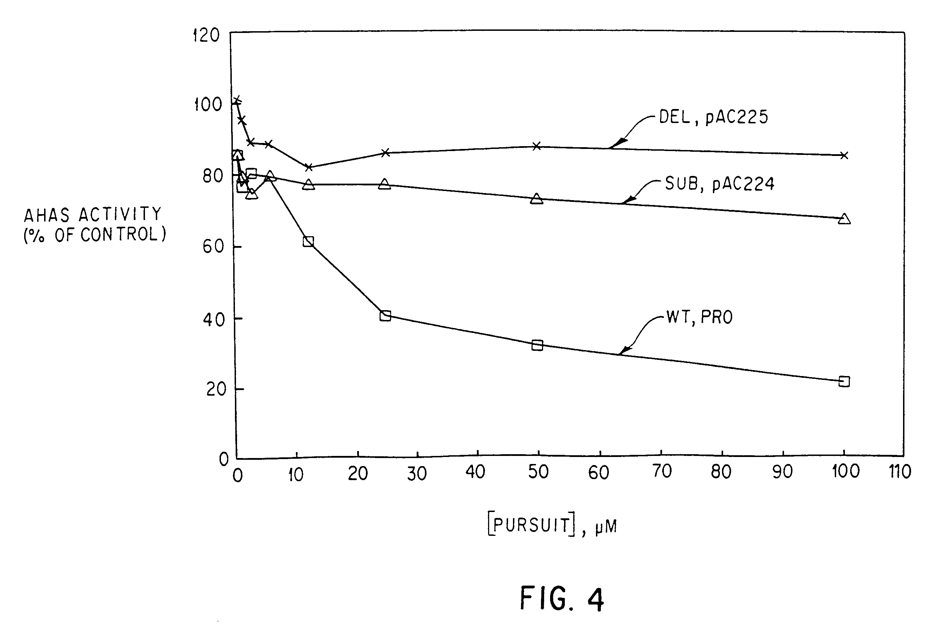

The results of these sprayings are depicted in Figure 4. The progeny data in the graph are presented with the most tolerant individual first and the most susceptible individual last for each herbicide rate. As can be seen from this graph and as would be expected from a selfed progeny of the original transgenic plant, the progeny are segregating for herbicide tolerance. Both susceptible progeny and individuals with varying degrees of herbicide tolerance can be observed.

The plant fresh weights (g) and means for each herbicide rate are presented in Table 2 with the mean fresh weights (g) summarized below (Table 1):

Table 1

| Tobacco Mean FW: (5 reps each) | Control | Transgenic Progeny |
|---|---|---|
| Herbicide Rate: | | |
| 0 g/ha | 52.1 | 53.7 |
| 10 g/ha | 8.1 | 36.4 |
| 20 g/ha | 2.0 | 28.8 |
| 40 g/ha | 0.8 | 20.9 |
| 80 g/ha | 0.6 | 26.5 |
| 160 g/ha | 0.2 | 21.4 |

As can been seen in this table, the selfed progeny of the transgenic tobacco plant (10-1) exhibit a high degree of tolerance to Pursuit® applied postemergence. The susceptible parental cultivar does not display herbicide tolerance at any of the rates tested.

Seeds resulting from transgenic plant 10-1 are also assayed for resistance to the imidazolinone Pursuit®. Seeds are plated onto medium containing Pursuit® at 0, 1.25, 2.5, 3.75, and 5.0 $\mu$M. Twenty seeds are plated per petri dish, with two dishes per treatment. Herbicide tolerance of seedlings is evaluated after three weeks. The results of the 5.0 $\mu$M treatment is presented in Table 2. Even at the lowest concentration (1.25 $\mu$M treatment), control plants exhibited herbicide sensitivity. These results illustrate inheritance of the herbicide resistant trait from parental plant 10-1 to its progeny.

Table 2

| Results of tobacco seed assay | | | | |
|---|---|---|---|---|
| Pursuit® Conc.(μM): 5 | | S* | D | R |
| Progeny: | W38 (wild-type) | 20 20 | 0 0 | 0 0 |
| | 10-1 Selfed | 7 3 | 6 11 | 6 6 |
| | 10-1 x 0 | 11 11 | 9 9 | 0 0 |
| | 0 x 10-1 | 10 8 | 9 8 | 0 0 |

* S = Susceptible; D = Damaged; R = Resistant

## EXAMPLE V

### AHAS enzyme assay in E. coli

**AHAS enzyme assay (expression in E. coli)** - The site directed mutants, pAC224 and pAC225, are utilized as templates for the generation of an E. coli based expression system. The insensitivity to herbicide is tested when the higher plant gene of interest is expressed in a bacterial strain devoid of any endogenous AHAS activity. To this end, the Nco1/Pst1 fragment of wild type Arabidopsis AHAS (pAC301), the Trp574-Leu substitution (pAC224) and the Trp574 deletion (pAC225) are subcloned into the Nco1/Pst1 site of the E. coli expression vector, pKK233-2 (purchased from Pharmacia). This expression plasmid contains an IPTG inducible promoter as well as a transcription termination sequence. Both regions surround an Nco1, HindIII and PstI linker sequence that allows proper orientation of the gene of interest in relation to the bacterial promoter. Subcloning and ligation of the respective NcoI/PstI fragments into NcoI/PstI digested pKK233-2 DNA (ampicillin resistance) is as previously described. The ligation reaction is transformed into the E. coli strain, MF2000 [ilvB800:mu-1, Bg132, ilvl15, thi-1, argE3, RPSL31, (ara-leu, ilvHI) 863, mtl-1, xyl-5 galK2, lacYl, recAl]. This bacterial strain is devoid of endogenous AHAS activity. Therefore, growth on a minimal media lacking isoleucine and valine (the end products of amino acid biosynthetic pathway of which the AHAS enzyme is involved) in an MF2000 strain transformed with either pAC224 (Trp574-Leu) or pAC225 (Trp del) requires the expression and function of the Arabidopsis gene in E. coli. Each of the three constructions complements MF2000 on agar media as well as in broth cultures.

Single colonies of MF2000 containing either the plasmid construction pAC210, pAC224 or pAC225 are inoculated into 3ml broth cultures of M63 minimal media (amp) containing the amino acids arginine, leucine, isoleucine and valine. [M63 is 30g $KH_3PO_4$, 70g $K_2HPO_4$, 20g $(NH_4)_2SO_4$, 5mg $FeSO_4$, 100ul of 1M $MgSO_4$, 200ul thiamine. The glucose concentration is raised to 1.2%]. The cultures are incubated overnight at 37°C with constant shaking. The bacterial cells are pelleted, resuspended in several mls of M63 (amp) supplemented with arginine and leucine, but lacking isoleucine and valine (minus ilv media). The cells are transferred to 10-50 ml of minus ilv media and constant shaking at 37°C was continued overnight. The cells are pelleted and either used directly in AHAS enzyme assays or frozen at -20°C until use.

The extraction and assay for Arabidopsis AHAS in bacterial cells is essentially as described by Singh, et. al. (J. Chromatography 444: 251-261, 1988). The bacterial pellet is powered in liquid nitrogen and homogenized in 100mM potassium phosphate buffer (pH 7.5) containing 10mM pyruvate, 5mM magnesium chloride, 5mM $Na_2$-EDTA, 100um flavin adenine dinucleotide (FAD), 1mM valine, 1mM leucine, 10%(v/v) glycerol and 10mM cysteine. The homogenate is filtered through a nylon cloth (53uM mesh) and centrifuged at 25,000 x g for 20 minutes. The supernatant fraction is brought to 50% saturation with respect to ammonium sulfate and allowed to stand for 20-30 minutes on ice. The precipitate is pelleted via centrifugation at 25,000 x g for 20 minutes. The supernatant is discarded and the ammonium sulfate pellet is used immediately or frozen with liquid nitrogen and stored at -20°C until use.

AHAS activity in the presence or absence of herbicide is measured by estimation of the product, acetolactate, after conversion by acid decarboxylation to acetoin. Standard reaction mixtures contain the enzyme (and herbicide) in 50mM potassium phosphate buffer (pH 7.0) containing 100mM sodium pyruvate, 10mM magnesium chloride, 1mM thiamine pyrophosphate (TPP) and 10uM FAD. This mixture is incubated at 37°C for 1 hr. The reaction is stopped with the addition of sulfuric acid to make a final concentration of 0.85%. The reaction product is allowed to decarboxylate at 60°C for 15 minutes. The acetoin formed is determined by incubating with creatine (0.17%) and 1-naphthol (1.7%) by the method of Westerfeld (J. Biol. Chem. 161: 495-502, 1945). Appropriate checks of direct acetoin formation during the enzyme assays are made.

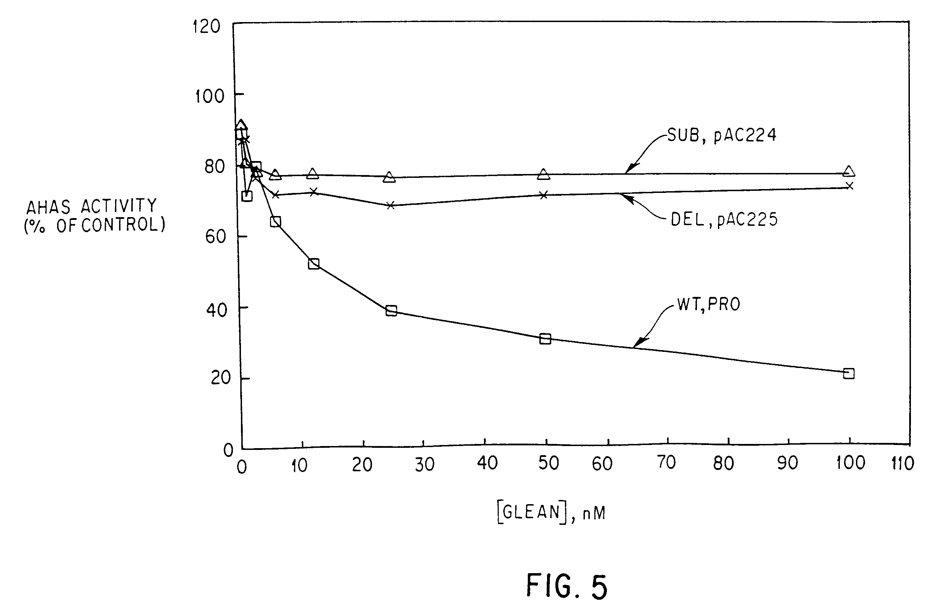

Wild type Arabidopsis AHAS (pAC201) is sensitive to the imidazolinone herbicide Pursuit® and the sulfonylurea herbicide Glean® whereas both the Trp574-Leu substitution (pAC224) and the Trp574 deletion (pAC225) are insensitive to increasing concentrations of Pursuit® herbicide (Figure 4) and Glean® herbicide (Figure 5). Surprisingly, the deletion confers a level of insensitivity to these herbicides which is equivalent or greater than that of the corresponding substitution. Therefore, deletion of the Trp574 residue of the Arabidopsis AHAS coding region results in a functional form of the enzyme that is insensitive to the addition of either of two unrelated herbicides capable of inhibiting the wild type form of the enzyme.

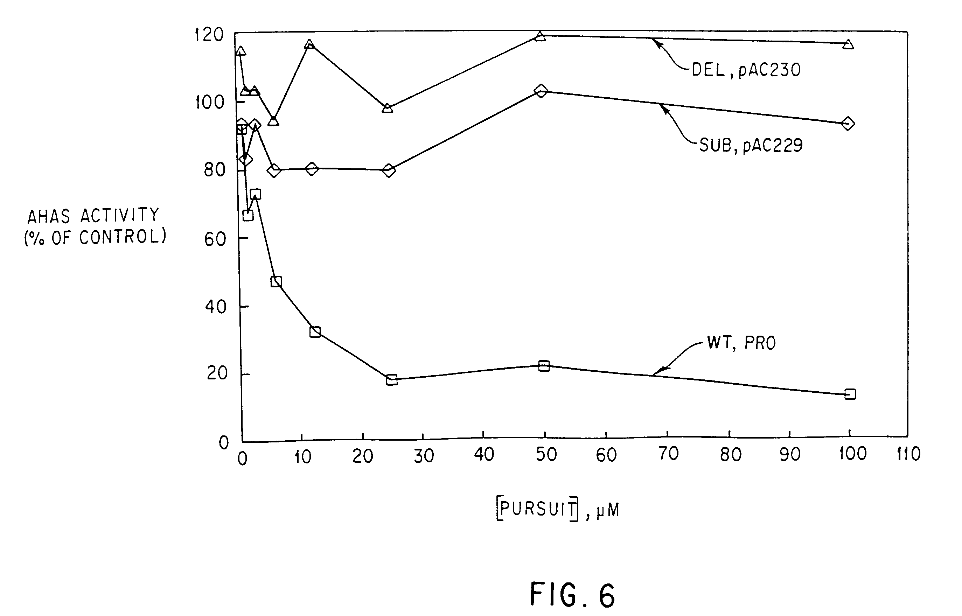

The procedures noted above are also used to create substitution of an asparagine for a serine at position 653, as well as a deletion at this position. Plasmids containing the substitution (pAC229) or the deletion (pAC230) are transformed into E. coli MF2000 and AHAS activity of deletion and substitution mutants in the presence of herbicides Pursuit® and Glean® is determined for each as described above. The AHAS produced by substitution of asparagine for serine is insensitive to inhibition by Pursuit® (Figure 6) as previously reported (Sathasivan et. al, supra), but sensitivity to Glean® was only slightly reduced (about 10%) as compared with the wild-type enzyme (Figure 7). In contrast, the AHAS produced by the Ser653 deletion is highly resistant to inhibition by both Pursuit® and Glean® (Figures 6 and 7).

**DEPOSIT OF BIOLOGICAL MATERIALS**

The following microorganisms were deposited with the American Type Culture Collection, 12301 Parklawn Drive, Rockville, Maryland, 20857, on December 6, 1990, and given accession numbers as follows:

| Microorganism | Accession No. |
|---|---|
| Agrobacterium strain LBA4404 harboring pAC351 (Agro/351) [Trp574 deletion] | 68488 |
| Agrobacterium strain LBA4404 harboring plasmid pAC324 [Ser653 deletion] | 68489 |

Sequence ID No.:  1

Sequence Type:  Amino Acid

Sequence Length:  670 Amino Acids

Strandedness:  Single

Topology:  Linear

Original Source Organism:  Arabidopsis

Properties:  acetohydroxyacid synthase enzyme


Met Ala Ala Ala Thr Thr Thr Thr Thr Thr Ser Ser
 1           5               10

Ser Ile Ser Phe Ser Thr Lys Pro Ser Pro Ser Ser
         15           20

Ser Lys Ser Pro Leu Pro Ile Ser Arg Phe Ser Leu
 25               30               35

Pro Phe Ser Leu Asn Pro Asn Lys Ser Ser Ser Ser
             40               45

Ser Arg Arg Arg Gly Ile Lys Ser Ser Ser Pro Ser
     50               55               60

Ser Ile Ser Ala Val Leu Asn Thr Thr Thr Asn Val
             65               70

Thr Thr Thr Pro Ser Pro Thr Lys Pro Thr Lys Pro
         75               80

Glu Thr Phe Ile Ser Arg Leu Ala Pro Asp Gln Pro
 85               90               95

Arg Lys Gly Ala Asp Ile Leu Val Glu Ala Leu Glu
             100              105

Arg Gln Gly Val Glu Thr Val Phe Ala Tyr Pro Gly
     110              115              120

Gly Ala Ser Met Glu Ile His Gln Ala Leu Thr Arg
             125                   130

Ser Ser Ser Ile Arg Asn Val Leu Pro Arg His Glu
         135              140

Gln Gly Gly Val Phe Ala Ala Glu Gly Tyr Ala Arg
 145              150              155

```
Ser Ser Gly Lys Pro Gly Ile Cys Ile Ala Thr Ser
            160                 165

Gly Pro Gly Ala Thr Asn Leu Val Ser Gly Leu Ala
            170             175             180

Asp Ala Leu Leu Asp Ser Val Pro Leu Val Ala Ile
                    185             190

Thr Gly Gln Val Pro Arg Arg Met Ile Gly Thr Asp
            195             200

Ala Phe Gln Glu Thr Pro Ile Val Glu Val Thr Arg
205                 210                 215

Ser Ile Thr Lys His Asn Tyr Leu Val Met Asp Val
            220                 225

Glu Asp Ile Pro Arg Ile Ile Glu Glu Ala Phe Phe
    230             235                 240

Leu Ala Thr Ser Gly Arg Pro Gly Pro Val Leu Val
                245             250

Asp Val Pro Lys Asp Ile Gln Gln Gln Leu Ala Ile
            255             260

Pro Asn Trp Glu Gln Ala Met Arg Leu Pro Gly Tyr
265                 270             275

Met Ser Arg Met Pro Lys Pro Pro Glu Asp Ser His
            280             285

Leu Glu Gln Ile Val Arg Leu Ile Ser Glu Ser Lys
    290             295                 300

Lys Pro Val Leu Tyr Val Gly Gly Gly Cys Leu Asn
                305             310

Ser Ser Asp Glu Leu Gly Arg Phe Val Glu Leu Thr
            315             320

Gly Ile Pro Val Ala Ser Thr Leu Met Gly Leu Gly
325                 330             335

Ser Tyr Pro Cys Asp Asp Glu Leu Ser Leu His Met
                340             345

Leu Gly Met His Gly Thr Val Tyr Ala Asn Tyr Ala
    350             355                 360

Val Glu His Ser Asp Leu Leu Leu Ala Phe Gly Val
                365             370
```

15

```
Arg Phe Asp Asp Arg Val Thr Gly Lys Leu Glu Ala
        375             380

Phe Ala Ser Arg Ala Lys Ile Val His Ile Asp Ile
385             390             395

Asp Ser Ala Glu Ile Gly Lys Asn Lys Thr Pro His
            400             405

Val Ser Val Cys Gly Asp Val Lys Leu Ala Leu Gln
    410             415             420

Gly Met Asn Lys Val Leu Glu Asn Arg Ala Glu Glu
            425             430

Leu Lys Leu Asp Phe Gly Val Trp Arg Asn Glu Leu
        435             440

Asn Val Gln Lys Gln Lys Phe Pro Leu Ser Phe Lys
445             450             455

Thr Phe Gly Glu Ala Ile Pro Pro Gln Tyr Ala Ile
            460             465

Lys Val Leu Asp Glu Leu Thr Asp Gly Lys Ala Ile
        470             475             480

Ile Ser Thr Gly Val Gly Gln His Gln Met Trp Ala
            485             490

Ala Gln Phe Tyr Asn Tyr Lys Lys Pro Arg Gln Trp
        495             500

Leu Ser Ser Gly Gly Leu Gly Ala Met Gly Phe Gly
505             510             515

Leu Pro Ala Ala Ile Gly Ala Ser Val Ala Asn Pro
            520             525

Asp Ala Ile Val Val Asp Ile Asp Gly Asp Gly Ser
    530             535             540

Phe Ile Met Asn Val Gln Glu Leu Ala Thr Ile Arg
            545             550

Val Glu Asn Leu Pro Val Lys Val Leu Leu Leu Asn
    555             560

Asn Gln His Leu Gly Met Val Met Gln Trp Glu Asp
565             570             575

Arg Phe Tyr Lys Ala Asn Arg Ala His Thr Phe Leu
        580             585
```

```
Gly Asp Pro Ala Gln Glu Asp Glu Ile Phe Pro Asn
    590                 595             600

Met Leu Leu Phe Ala Ala Ala Cys Gly Ile Pro Ala
            605                 610

Ala Arg Val Thr Lys Lys Ala Asp Leu Arg Glu Ala
        615             620

Ile Gln Thr Met Leu Asp Thr Pro Gly Pro Tyr Leu
625             630                 635

Leu Asp Val Ile Cys Pro His Gln Glu His Val Leu
            640                 645

Pro Met Ile Pro Ser Gly Gly Thr Phe Asn Asp Val
    650             655             660

Ile Thr Glu Gly Asp Gly Arg Ile Lys Tyr
            665             670
```

Sequence ID No.:  2

Sequence Type:  Nucleic Acid

Sequence Length:  2,526 Base Pairs

Strandedness:  Single

Topology:  Linear

Original Source Organism:  <u>Arabidopsis</u>

Properties:  gene encoding acetohydroxyacid synthase
enzyme

| | | | | |
|---|---|---|---|---|
| NTRAAARAAA | GAAAGAAAGA | TCAATTTGAT | AAATTTCTCA | 40 |
| GCCACAAATT | CTACATTTAG | GTTTTAGCAT | ATCGAAGGCT | 80 |
| CAATCACAAA | TACAATAGAT | AGACTAGAGA | TTCCAGCGTC | 120 |
| ACGTGAGTTT | TATCTATAAA | TAAAGGACCA | AAAATCAAAT | 160 |
| CCCGAGGGCA | TTTTCGTAAT | CCAACATAAA | ACCCTTAAAC | 200 |
| TTCAAGTCTC | ATTTTTAAAC | AAATCATGTT | CACAAGTCTC | 240 |
| TTCTTCTTCT | CTGTTTCTCT | ATCTCTTGCT | CATCTTTCTC | 280 |
| CTGAACCATG | GCGGCGGCAA | CAACAACAAC | AACAACATCT | 320 |
| TCTTCGATCT | CCTTCTCCAC | CAAACCATCT | CCTTCCTCCT | 360 |
| CCAAATCACC | ATTACCAATC | TCCAGATTCT | CCCTCCCATT | 400 |
| CTCCCTAAAC | CCCAACAAAT | CATCCTCCTC | CTCCCGCCGC | 440 |
| CGCGGTATCA | AATCCAGCTC | TCCCTCCTCC | ATCTCCGCCG | 480 |
| TGCTCAACAC | AACCACCAAT | GTCACAACCA | CTCCCTCTCC | 520 |
| AACCAAACCT | ACCAAACCCG | AAACATTCAT | CTCCCGATTG | 560 |
| GCTCCAGATC | AACCCCGCAA | AGGCGCTGAT | ATCCTCGTCG | 600 |
| AAGCTTTAGA | ACGTCAAGGC | GTAGAAACCG | TATTCGCTTA | 640 |
| CCCTGGAGGT | GCATCAATGG | AGATTCACCA | AGCCTTAACC | 680 |
| CGCTCTTCCT | CAATCCGTAA | CGTCCTTCCT | CGTCACGAAC | 720 |
| AAGGAGGTGT | ATTCGCAGCA | GAAGGATACG | CTCGATCCTC | 760 |
| AGGTAAACCA | GGTATCTGTA | TAGCCACTTC | AGGTCCCGGA | 800 |

```
GCTACAAATC TCGTTAGCGG ATTAGCCGAT GCGTTGTTAG        840

ATAGTGTTCC TCTTGTAGCA ATCACAGGAC AAGTCCCTCG        880

TCGTATGATT GGTACAGATG CGTTTCAAGA GACTCCGATT        920

GTTGAGGTAA CGCGTTCGAT TACGAAGCAT AACTATCTTG        960

TGATGGATGT TGAAGATATC CCTAGGATTA TTGAGGAAGC       1000

TTTCTTTTTA GCTACTTCTG GTAGACCTGG ACCTGTTTTG       1040

GTTGATGTTC CTAAAGATAT TCAACAACAG CTTGCGATTC       1080

CTAATTGGGA ACAGGCTATG AGATTACCTG GTTATATGTC       1120

TAGGATGCCT AAACCTCCGG AAGATTCTCA TTTGGAGCAG       1160

ATTGTTAGGT TGATTTCTGA GTCTAAGAAG CCTGTGTTGT       1200

ATGTTGGTGG TGGTTGTTTG AATTCTAGCG ATGAATTGGG       1240

TAGGTTTGTT GAGCTTACGG GGATCCCTGT TGCGAGTACG       1280

TTGATGGGGC TGGGATCTTA TCCTTGTGAT GATGAGTTGT       1320

CGTTACATAT GCTTGGAATG CATGGGACTG TGTATGCAAA       1360

TTACGCTGTG GAGCATAGTG ATTTGTTGTT GGCGTTTGGG       1400

GTAAGGTTTG ATGATCGTGT CACGGGTAAG CTTGAGGCTT       1440

TTGCTAGTAG GGCTAAGATT GTTCATATTG ATATTGACTC       1480

GGCTGAGATT GGGAAGAATA AGACTCCTCA TGTGTCTGTG       1520

TGTGGTGATG TTAAGCTGGC TTTGCAAGGG ATGAATAAGG       1560

TTCTTGAGAA CCGAGCGGAG GAGCTTAAGC TTGATTTTGG       1600

AGTTTGGAGG AATGAGTTGA ACGTACAGAA ACAGAAGTTT       1640

CCGTTGAGCT TTAAGACGTT TGGGGAAGCT ATTCCTCCAC       1680

AGTATGCGAT TAAGGTCCTT GATGAGTTGA CTGATGGAAA       1720

AGCCATAATA AGTACTGGTG TCGGGCAACA TCAAATGTGG       1760

GCGGCGCAGT TCTACAATTA CAAGAAACCA AGGCAGTGGC       1800

TATCATCAGG AGGCCTTGGA GCTATGGGAT TTGGACTTCC       1840

TGCTGCGATT GGAGCGTCTG TTGCTAACCC TGATGCGATA       1880
```

```
GTTGTGGATA TTGACGGAGA TGGAAGCTTT ATAATGAATG          1920

TGCAAGAGCT AGCCACTATT CGTGTAGAGA ATCTTCCAGT          1960

GAAGGTACTT TTATTAAACA ACCAGCATCT TGGCATGGTT          2000

ATGCAATGGG AAGATCGGTT CTACAAAGCT AACCGAGCTC          2040

ACACATTTCT CGGGGATCCG GCTCAGGAGG ACGAGATATT          2080

CCCGAACATG TTGCTGTTTG CAGCAGCTTG CGGGATTCCA          2120

GCGGCGAGGG TGACAAAGAA AGCAGATCTC CGAGAAGCTA          2160

TTCAGACAAT GCTGGATACA CCAGGACCTT ACCTGTTGGA          2200

TGTGATTTGT CCGCACCAAG AACATGTGTT GCCGATGATC          2240

CCGAGTGGTG GCACTTTCAA CGATGTCATA ACGGAAGGAG          2280

ATGGCCGGAT TAAATACTGA GAGATGAAAC CGGTGATTAT          2320

CAGAACCTTT TATGGTCTTT GTATGCATAT GGTAAAAAAA          2360

CTTAGTTTGC AATTTCCTGT TTGTTTTGGT AATTTGAGTT          2400

TCTTTTAGTT GTTGATCTGC CTGCTTTTTG GTTTACGTCA          2440

GACTACTACT GCTGTTGTTG TTTGGTTTCC TTTCTTTCAT          2480

TTTATAAATA AATAATCCGG TTCGGTTTAC TCCTTGTGAC          2520

TGGCTC                                              2526
```

## Claims

1. A method for expressing an herbicide resistant AHAS enzyme in a host cell comprising transforming the host cell with a nucleic acid sequence encoding a functional AHAS enzyme, which enzyme has an amino acid sequence differing from a wild-type AHAS sequence in having at least one amino acid deletion that confers herbicide resistance to the enzyme, and culturing the host cell under conditions permitting expression of the enzyme.

2. The method of Claim 1 in which a deletion is present in a conserved region of the AHAS sequence.

3. The method of any of Claims 1-2 in which a deletion has occurred in a region of the wild type AHAS sequence Arabidopsis selected from the group consisting of amino acids 119-122, 194-197, 201-208, 255-257, 348-353, 373-377, 569-578, and 650-653, or the homologous regions in a different species.

4. The method of Claims 1-3 in which the deletion occurs at at least one position selected from the group consisting of amino acids 121, 122, 197, 205, 256, 351, 376, 571, 574, 578 and 653.

5. The method of any of Claims 1-4 in which resistance is to at least one herbicide selected from the group consisting of an imidazolinone, a sulfonylurea and a triazolopyrimidine.

20

6. The method of any of Claims 1-5 in which the host cell is a plant cell.

7. The method of Claim 6 in which the transformed plant cell is regenerated to a mature plant expressing an herbicide resistant phenotype.

8. A method for producing a vector useful in conferring herbicide resistance to a host cell comprising ligating into an appropriate vector a nucleic acid sequence encoding a functional AHAS enzyme, which enzyme has an amino acid sequence differing from a wild type AHAS in having at least one amino acid deletion that confers herbicide resistance to the enzyme.

9. A method for conferring herbicide resistance to a plant cell which comprises providing the plant cell with a nucleic acid sequence encoding a functional AHAS enzyme, which enzyme has an amino acid sequence differing from a wild type AHAS sequence in having at least one amino acid deletion that confers herbicide resistance to the enzyme.

10. A method for growing herbicide resistant plants which comprises cultivating a plant which produces a functional AHAS enzyme with an amino acid sequence differing from a wild type AHAS sequence in having at least one amino acid deletion that confers herbicide resistance to the enzyme, in the presense of an inhibitory amount of the herbicide.

11. A method of selecting host cells successfully transformed with a gene of interest which comprises providing to prospective host cells the gene of interest linked to a nucleic acid sequence encoding a functional AHAS enzyme, which enzyme has an amino acid sequence differing from a wild-type AHAS sequence in having at least one amino acid deletion that confers herbicide resistance to the enzyme, and culturing the host cell under conditions permitting expression of the enzyme, growing the cells in the presence of an inhibitory amount of a herbicide, and identifying surviving cells as containing the gene of interest.

```
Met Ala Ala Ala Thr Thr Thr Thr Thr Thr Ser Ser
 1               5                  10

Ser Ile Ser Phe Ser Thr Lys Pro Ser Pro Ser Ser
        15              20

Ser Lys Ser Pro Leu Pro Ile Ser Arg Phe Ser Leu
25                  30                  35

Pro Phe Ser Leu Asn Pro Asn Lys Ser Ser Ser Ser
            40              45

Ser Arg Arg Arg Gly Ile Lys Ser Ser Ser Pro Ser
    50                  55                      60

Ser Ile Ser Ala Val Leu Asn Thr Thr Thr Asn Val
                65              70

Thr Thr Thr Pro Ser Pro Thr Lys Pro Thr Lys Pro
        75                  80

Glu Thr Phe Ile Ser Arg Leu Ala Pro Asp Gln Pro
85                  90                  95

Arg Lys Gly Ala Asp Ile Leu Val Glu Ala Leu Glu
            100                 105

Arg Gln Gly Val Glu Thr Val Phe Ala Tyr Pro Gly
    110                 115                 120

Gly Ala Ser Met Glu Ile His Gln Ala Leu Thr Arg
                125                 130

Ser Ser Ser Ile Arg Asn Val Leu Pro Arg His Glu
        135                 140

Gln Gly Gly Val Phe Ala Ala Glu Gly Tyr Ala Arg
145                 150                 155

Ser Ser Gly Lys Pro Gly Ile Cys Ile Ala Thr Ser
            160                 165

Gly Pro Gly Ala Thr Asn Leu Val Ser Gly Leu Ala
    170                 175                 180

Asp Ala Leu Leu Asp Ser Val Pro Leu Val Ala Ile
                185                 190

Thr Gly Gln Val Pro Arg Arg Met Ile Gly Thr Asp
        195                 200

Ala Phe Gln Glu Thr Pro Ile Val Glu Val Thr Arg
205                 210                 215
```

## FIG.Ia(i)

Ser Ile Thr Lys His Asn Tyr Leu Val Met Asp Val
            220                 225

Glu Asp Ile Pro Arg Ile Ile Glu Glu Ala Phe Phe
    230             235                     240

Leu Ala Thr Ser Gly Arg Pro Gly Pro Val Leu Val
                245                 250

Asp Val Pro Lys Asp Ile Gln Gln Gln Leu Ala Ile
             255             260

Pro Asn Trp Glu Gln Ala Met Arg Leu Pro Gly Tyr
265                 270                 275

Met Ser Arg Met Pro Lys Pro Pro Glu Asp Ser His
            280                 285

Leu Glu Gln Ile Val Arg Leu Ile Ser Glu Ser Lys
    290                 295                 300

Lys Pro Val Leu Tyr Val Gly Gly Gly Cys Leu Asn
                305                 310

Ser Ser Asp Glu Leu Gly Arg Phe Val Glu Leu Thr
        315                 320

Gly Ile Pro Val Ala Ser Thr Leu Met Gly Leu Gly
325                 330                 335

Ser Tyr Pro Cys Asp Asp Glu Leu Ser Leu His Met
            340                 345

Leu Gly Met His Gly Thr Val Tyr Ala Asn Tyr Ala
    350                 355                 360

Val Glu His Ser Asp Leu Leu Leu Ala Phe Gly Val
            365                 370

Arg Phe Asp Asp Arg Val Thr Gly Lys Leu Glu Ala
    375                 380

Phe Ala Ser Arg Ala Lys Ile Val His Ile Asp Ile
385                 390                 395

Asp Ser Ala Glu Ile Gly Lys Asn Lys Thr Pro His
            400                 405

Val Ser Val Cys Gly Asp Val Lys Leu Ala Leu Gln
    410             415                     420

Gly Met Asn Lys Val Leu Glu Asn Arg Ala Glu Glu
            425                 430

# FIG. Ia (ii)

23

```
Leu Lys Leu Asp Phe Gly Val Trp Arg Asn Glu Leu
        435             440

Asn Val Gln Lys Gln Lys Phe Pro Leu Ser Phe Lys
445             450             455

Thr Phe Gly Glu Ala Ile Pro Pro Gln Tyr Ala Ile
        460             465

Lys Val Leu Asp Glu Leu Thr Asp Gly Lys Ala Ile
    470             475             480

Ile Ser Thr Gly Val Gly Gln His Gln Met Trp Ala
            485             490

Ala Gln Phe Tyr Asn Tyr Lys Lys Pro Arg Gln Trp
        495             500

Leu Ser Ser Gly Gly Leu Gly Ala Met Gly Phe Gly
505             510             515

Leu Pro Ala Ala Ile Gly Ala Ser Val Ala Asn Pro
        520             525

Asp Ala Ile Val Val Asp Ile Asp Gly Asp Gly Ser
    530             535             540

Phe Ile Met Asn Val Gln Glu Leu Ala Thr Ile Arg
            545             550

Val Glu Asn Leu Pro Val Lys Val Leu Leu Leu Asn
        555             560

Asn Gln His Leu Gly Met Val Met Gln Trp Glu Asp
565             570             575

Arg Phe Tyr Lys Ala Asn Arg Ala His Thr Phe Leu
        580             585

Gly Asp Pro Ala Gln Glu Asp Glu Ile Phe Pro Asn
    590             595             600

Met Leu Leu Phe Ala Ala Ala Cys Gly Ile Pro Ala
            605             610

Ala Arg Val Thr Lys Lys Ala Asp Leu Arg Glu Ala
        615             620

Ile Gln Thr Met Leu Asp Thr Pro Gly Pro Tyr Leu
625             630             635

Leu Asp Val Ile Cys Pro His Gln Glu His Val Leu
        640             645
```

# FIG.la(iii)

Pro Met Ile Pro Ser Gly Gly Thr Phe Asn Asp Val
    650                   655               660

Ile Thr Glu Gly Asp Gly Arg Ile Lys Tyr
              665               670

# FIG.Ia(iiii)

```
NTRAAARAAA  GAAAGAAAGA  TCAATTTGAT  AAATTTCTCA          40

GCCACAAATT  CTACATTTAG  GTTTTAGCAT  ATCGAAGGCT          80

CAATCACAAA  TACAATAGAT  AGACTAGAGA  TTCCAGCGTC         120

ACGTGAGTTT  TATCTATAAA  TAAAGGACCA  AAAATCAAAT         160

CCCGAGGGCA  TTTTCGTAAT  CCAACATAAA  ACCCTTAAAC         200

TTCAAGTCTC  ATTTTTAAAC  AAATCATGTT  CACAAGTCTC         240

TTCTTCTTCT  CTGTTTCTCT  ATCTCTTGCT  CATCTTTCTC         280

CTGAACCATG  GCGGCGGCAA  CAACAACAAC  AACAACATCT         320
        ↑
TCTTCGATCT  CCTTCTCCAC  CAAACCATCT  CCTTCCTCCT         360

CCAAATCACC  ATTACCAATC  TCCAGATTCT  CCCTCCCATT         400

CTCCCTAAAC  CCCAACAAAT  CATCCTCCTC  CTCCCGCCGC         440

CGCGGTATCA  AATCCAGCTC  TCCCTCCTCC  ATCTCCGCCG         480

TGCTCAACAC  AACCACCAAT  GTCACAACCA  CTCCCTCTCC         520

AACCAAACCT  ACCAAACCCG  AAACATTCAT  CTCCCGATTG         560

GCTCCAGATC  AACCCCGCAA  AGGCGCTGAT  ATCCTCGTCG         600

AAGCTTTAGA  ACGTCAAGGC  GTAGAAACCG  TATTCGCTTA         640

CCCTGGAGGT  GCATCAATGG  AGATTCACCA  AGCCTTAACC         680

CGCTCTTCCT  CAATCCGTAA  CGTCCTTCCT  CGTCACGAAC         720

AAGGAGGTGT  ATTCGCAGCA  GAAGGATACG  CTCGATCCTC         760

AGGTAAACCA  GGTATCTGTA  TAGCCACTTC  AGGTCCCGGA         800

GCTACAAATC  TCGTTAGCGG  ATTAGCCGAT  GCGTTGTTAG         840

ATAGTGTTCC  TCTTGTAGCA  ATCACAGGAC  AAGTCCCTCG         880

TCGTATGATT  GGTACAGATG  CGTTTCAAGA  GACTCCGATT         920

GTTGAGGTAA  CGCGTTCGAT  TACGAAGCAT  AACTATCTTG         960

TGATGGATGT  TGAAGATATC  CCTAGGATTA  TTGAGGAAGC        1000

TTTCTTTTTA  GCTACTTCTG  GTAGACCTGG  ACCTGTTTTG        1040

GTTGATGTTC  CTAAAGATAT  TCAACAACAG  CTTGCGATTC        1080
```

# FIG.Ib(i)

26

CTAATTGGGA ACAGGCTATG AGATTACCTG GTTATATGTC          1120

TAGGATGCCT AAACCTCCGG AAGATTCTCA TTTGGAGCAG          1160

ATTGTTAGGT TGATTTCTGA GTCTAAGAAG CCTGTGTTGT          1200

ATGTTGGTGG TGGTTGTTTG AATTCTAGCG ATGAATTGGG          1240

TAGGTTTGTT GAGCTTACGG GGATCCCTGT TGCGAGTACG          1280

TTGATGGGGC TGGGATCTTA TCCTTGTGAT GATGAGTTGT          1320

CGTTACATAT GCTTGGAATG CATGGGACTG TGTATGCAAA          1360

TTACGCTGTG GAGCATAGTG ATTTGTTGTT GGCGTTTGGG          1400

GTAAGGTTTG ATGATCGTGT CACGGGTAAG CTTGAGGCTT          1440

TTGCTAGTAG GGCTAAGATT GTTCATATTG ATATTGACTC          1480

GGCTGAGATT GGGAAGAATA AGACTCCTCA TGTCTCTGTG          1520

TGTGGTGATG TTAAGCTGGC TTTGCAAGGG ATGAATAAGG          1560

TTCTTGAGAA CCGAGCGGAG GAGCTTAAGC TTGATTTTGG          1600

AGTTTGGAGG AATGAGTTGA ACGTACAGAA ACAGAAGTTT          1640

CCGTTGAGCT TTAAGACGTT TGGGGAAGCT ATTCCTCCAC          1680

AGTATGCGAT TAAGGTCCTT GATGAGTTGA CTGATGGAAA          1720

AGCCATAATA AGTACTGGTG TCGGGCAACA TCAAATGTGG          1760

GCGGCGCAGT TCTACAATTA CAAGAAACCA AGGCAGTGGC          1800

TATCATCAGG AGGCCTTGGA GCTATGGGAT TTGGACTTCC          1840

TGCTGCGATT GGAGCGTCTG TTGCTAACCC TGATGCGATA          1880

GTTGTGGATA TTGACGGAGA TGGAAGCTTT ATAATGAATG          1920

TGCAAGAGCT AGCCACTATT CGTGTAGAGA ATCTTCCAGT          1960

GAAGGTACTT TTATTAAACA ACCAGCATCT TGGCATGGTT          2000

ATGCAATGGG AAGATCGGTT CTACAAAGCT AACCGAGCTC          2040

ACACATTTCT CGGGGATCCG GCTCAGGAGG ACGAGATATT          2080

CCCGAACATG TTGCTGTTTG CAGCAGCTTG CGGGATTCCA          2120

GCGGCGAGGG TGACAAAGAA AGCAGATCTC CGAGAAGCTA          2160

# FIG.Ib(ii)

TTCAGACAAT GCTGGATACA CCAGGACCTT ACCTGTTGGA 2200

TGTGATTTGT CCGCACCAAG AACATGTGTT GCCGATGATC 2240

CCGAGTGGTG GCACTTTCAA CGATGTCATA ACGGAAGGAG 2280

ATGGCCGGAT TAAATACTGA GAGATGAAAC CGGTGATTAT 2320

CAGAACCTTT TATGGTCTTT GTATGCATAT GGTAAAAAAA 2360

CTTAGTTTGC AATTTCCTGT TTGTTTTGGT AATTTGAGTT 2400

TCTTTTAGTT GTTGATCTGC CTGCTTTTTG GTTTACGTCA 2440

GACTACTACT GCTGTTGTTG TTTGGTTTCC TTTCTTTCAT 2480

TTTATAAATA AATAATCCGG TTCGGTTTAC TCCTTGTGAC 2520

TGGCTC 2526

# FIG.Ib(iii)

FIG.2a

FIG. 2b

FIG.2c

FIG. 3

EP 0 492 113 A2

FIG. 4

EP 0 492 113 A2

FIG. 5

FIG. 6

FIG. 7

EP 0 492 113 A2

FIG. 8